# EUROPEAN PATENT SPECIFICATION

(11) **EP 4 608 241 B1**
(45) Date of publication and mention of the grant of the patent: **30.09.2026**
(21) Application number: 23805828.3
(22) Date of filing: 19.10.2023
(51) Int. Cl.: A61B 5/00, A61N 1/05, A61N 1/36

(54) **LEAD FIXATION ELEMENT FOR OBSTRUCTIVE SLEEP APNEA**
LEITUNGSFIXIERUNGSELEMENT FÜR OBSTRUKTIVE SCHLAFAPNOE
ÉLÉMENT DE FIXATION D'IMPLANT POUR L'APNÉE OBSTRUCTIVE DU SOMMEIL

(30) Priority: 27.10.2022 US 202263381239 P
(43) Date of publication of application: 03.09.2025
(73) Proprietor: Medtronic, Inc., Minneapolis, Minnesota 55432 (US)
(72) Inventor: SCHEINER, Avram, Mounds View, Minnesota 55112 (US); FALKNER, Phillip, C., Minneapolis, Minnesota 55432-3568 (US)
(74) Representative: Zimmermann & Partner Patentanwälte mbB
(86) International application number: PCT/US2023/077302
(87) International publication number: WO 2024/091837

(56) References cited:
- WO-A1-2021/242633
- US-A1- 2015 374 991
- US-A1- 2019 351 218
- US-A1- 2021 290 957

## Description

This application claims the benefit of U.S. Provisional Patent Application Serial No. 63/381,239, filed October 27, 2022, and entitled "LEAD FIXATION ELEMENT FOR OBSTRUCTIVE SLEEP APNEA.

### TECHNICAL FIELD

This disclosure relates to medical device systems and, more particularly, to medical device systems for delivery of electrical stimulation therapy.

### BACKGROUND

Obstructive sleep apnea (OSA), which encompasses apnea and hypopnea, is a disorder in which breathing may be irregularly and repeatedly stopped and started during sleep, resulting in disrupted sleep and reduced blood oxygen levels. Muscles in a patient's throat intermittently relax thereby allowing soft tissues of the throat to obstruct the upper airway while sleeping and cause OSA. In patients with a smaller than normal airway, airflow into the upper airway can be obstructed by the tongue or soft pallet moving to the back of the throat and covering the airway. Loss of air flow also causes unusual inter-thoracic pressure as a person tries to breathe with a blocked airway. Lack of adequate levels of oxygen during sleep can contribute to abnormal heart rhythms, heart attack, heart failure, high blood pressure, stroke, memory problems, and increased accidents during the day due to inadequate sleep. Additionally, loss of sleep occurs when a person is awakened during an apneic episode. Documents WO 2021/242633 A1, US 2021/290957 A1, US 2015/374991 A1 and US 2019/351217 A1 relate to neurostimulation devices.

### SUMMARY

The invention is defined in the appended claims. The devices, systems, and techniques of this disclosure generally relate to an implantable medical device (IMD) system and methods for therapy for obstructive sleep apnea (OSA) but can be extended to address other patient symptoms and disorders. With OSA, a patient's tongue may relax during sleep and block the patient's airway. Some example techniques to address OSA include electrically stimulating one or both hypoglossal nerves and/or motor points in the tongue of the patient. In response to the electrical stimulation, the hypoglossal nerve(s) causes protrusor muscles (e.g., genioglossus and geniohyoid muscles) to contract and move the tongue forward, thereby opening the airway.
In some examples, in response to stimulating at the motor points of the protrusor muscles (e.g., a location where an axon of the hypoglossal nerve terminates at a muscle fiber), the protrusor muscles may contract to move the tongue forward, thereby opening the airway.

To stimulate the hypoglossal nerve(s) and/or motor points, a medical device outputs electrical stimulation therapy via one or more electrodes on one or more implanted leads to cause the tongue to move forward. A medical professional can implant the one or more leads into the tongue of the patient using a needle. The one or more implanted leads each include one or more electrodes coupled to the medical device (e.g., an implantable or external medical device that delivers electrical stimulation via one or more electrodes on the lead).

To place a lead into the tongue of the patient, the medical professional may insert a needle into the tissue of the patient and near a target area (e.g., the hypoglossal nerve(s) and/or motor points). The medical professional may then insert a guidewire into the tissue of the patient through an inner lumen of the needle and remove the needle once the guidewire is in place. The medical professional may then advance an introducer sheath over the guidewire, place the lead in an inner lumen of the introducer sheath, advance the lead through the introducer sheath, and remove the introducer sheath once the lead is in place. In some examples, rather than using an introducer sheath, the medical professional may insert the lead into the lumen of the needle, and then remove the lead.

The lead is fixed with tissue of the patient prior to the removal of the introducer sheath and/or needle to prevent damage and/or dislodgement of the lead, e.g., in response to movement of the tongue of the patient. Some leads include fixation mechanisms (e.g., tines) located proximal to the one or more electrodes (e.g., away from the distal end of the lead, and towards the medical device) and, when deployed, extend away from the elongated body of the lead to secure the lead to tissue of the patient. The proximal placement of the fixation mechanisms may lead to dislodgement of the lead in case of inadequate engagement with the tissue. In addition, a clinician may have difficulty removing the lead from and/or repositioning the lead within the tissue due to the fixation mechanisms extending away from the elongated body.

This disclosure describes example fixation mechanisms for implantable leads to simplify fixing the implantable lead within the tissue of the patient. In addition, the example fixation mechanisms described herein may simplify repositioning of the implantable lead within the tissue. Although the example techniques are described with respect to lead placement in the tongue for treating OSA, the example techniques should not be considered to be limited to lead placement in the tongue or limited to treating OSA.

In one example, the disclosure describes a system according to the claimed invention comprising: an introducer configured to navigate a lead for placement near a hypoglossal nerve of a patient, the introducer comprising an elongated body defining an introducer lumen; and a lead configured to be disposed within the introducer lumen of the elongated body, the lead comprising: an elongated shaft defining a longitudinal axis; one or more electrodes disposed on a distal portion of the shaft, the one or more electrodes being configured to be placed near the hypoglossal nerve and configured to stimulate the hypoglossal nerve for treating obstructive sleep apnea (OSA); and a fixation element disposed over the distal portion of the elongated shaft, the fixation element comprising: a distal tip configured to penetrate tissue near the hypoglossal nerve; and a helix comprising a plurality of coils, the plurality of coils being configured to engage the tissue near the hypoglossal nerve to secure the lead near the hypoglossal nerve, wherein the lead further comprises a plurality of protrusions extending from an outer surface of the elongated shaft, wherein the plurality of protrusions are configured to engage with the tissue of the patient and prevent rotation of the fixation element within the tissue.

In another example, the disclosure describes a method comprising: advancing a lead within an introducer to a location within tissue of a patient near a hypoglossal nerve of the patient, the lead comprising: an elongated shaft defining a longitudinal axis; and one or more electrodes disposed on a distal portion of the shaft, wherein the lead is disposed within an introducer lumen defined by an elongated body of the introducer; and a fixation element disposed on the distal portion of the elongated shaft; puncturing the tissue near the hypoglossal nerve via a distal tip of the fixation element; advancing a helix of the fixation element distally into the tissue, wherein the helix comprises a plurality of coils; placing the one or more electrodes near the hypoglossal nerve; and retracting the catheter proximally away from the distal portion of the elongated shaft.

In another example, the disclosure describes an implantable medical lead configured to be placed near a hypoglossal nerve of a patient, the lead comprising: an elongated shaft defining a longitudinal axis; one or more electrodes disposed on a distal portion of the elongated shaft, the one or more electrodes being configured to be placed near the hypoglossal nerve and configured to stimulate the hypoglossal nerve for treating obstructive sleep apnea (OSA); and a fixation element disposed on the distal portion of the elongated shaft, the fixation element comprising: a distal tip configured to penetrate into tissue near the hypoglossal nerve; and a helix having a plurality of coils, the plurality of coils being configured to engage the tissue near the hypoglossal nerve to secure the lead near the hypoglossal nerve.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a conceptual diagram of an implantable medical device (IMD) system for delivering obstructive sleep apnea (OSA) therapy.
FIG. 2 is a conceptual diagram illustrating example locations of motor points where stimulation for OSA therapy may be delivered.
FIG. 3 is a block diagram illustrating example configurations of implantable medical devices (IMDs) which may be utilized in the system of FIG. 1.
FIG. 4 is a block diagram illustrating an example configuration of an external programmer.
FIG. 5A is a conceptual diagram illustrating an example implantable lead with a fixation element.
FIG. 5B is a conceptual diagram illustrating a distal portion of the example implantable lead of FIG. 5A.
FIG. 5C is a conceptual diagram illustrating a cross-sectional view of the distal portion of the example implantable lead of FIG. 5A, the cross section being taken along line A-A in FIG. 5B.
FIG. 5D is a conceptual diagram illustrating another example distal portion of the example implantable lead of FIG. 5A.
FIG. 6A is a conceptual diagram illustrating an example distal portion of the example implantable lead with an electrically connected fixation element.
FIG. 6B is a conceptual diagram illustrating an example distal portion of the example implantable lead with another example electrically connected fixation element.
FIG. 7 is a conceptual diagram illustrating the example implantable lead of FIG. 5A with an example guide sheath.
FIG. 8A is a conceptual diagram illustrating an example implantable lead with another example fixation element.
FIG. 8B is a conceptual diagram illustrating the example distal portion of the example implantable lead of FIG. 8A.
FIG. 9 is a flowchart illustrating an example method of implanting an example implantable lead near a hypoglossal nerve(s) of a patient.

### DETAILED DESCRIPTION

Medical devices, systems, and techniques for delivering electrical stimulation to the protrusor muscles of the tongue for the treatment of obstructive sleep apnea (OSA) are described in this disclosure. Electrical stimulation is delivered to cause the tongue of a patient to enter an advanced state, during sleep, to avoid or reduce upper airway obstruction. As used herein, the term, "advanced state" with regard to the tongue refers to a position that is moved forward and/or downward compared to a non-stimulated position or a relaxed position of the tongue. The advanced state is a state associated with contraction (e.g., via innervation from nerves in response to electrical stimulation) of protrusor muscles of the tongue (also sometimes referred to as "protruder" muscles of the tongue) including the genioglossus and geniohyoid muscles. An advanced state may be the opposite of a retracted and/or elevated position associated with the contraction of the retractor muscles (e.g., styloglossus and hyoglossus muscles) which retract and elevate the tongue. Electrical stimulation is delivered to cause the tongue to move (e.g., by depolarizing the nerve(s) that innervate the genioglossus and/or geniohyoid muscles) and maintain an advanced state. As discussed above, the advanced state may prevent collapse or blockage of, open, or widen the upper airway of a patient to at least partially maintain or increase airflow (e.g., promote unrestricted airflow or at least reduced restriction of airflow during breathing).

A surgeon implants one or more leads that each include one or electrodes into the tongue such that the electrodes are proximate to a hypoglossal nerve and/or motor points (e.g., one or more locations where axons of the hypoglossal nerve terminate at respective muscle fibers of the protrusor muscles). For example, there are two hypoglossal nerves in the tongue of the patient. In one example, one lead may be used to stimulate (e.g., by delivering electrical stimulation through one or more electrodes of the lead) one of the two hypoglossal nerves, one lead may be used to stimulate both hypoglossal nerves, or two leads may be used, where each lead stimulates a respective one of the hypoglossal nerves. Stimulation of either or both hypoglossal nerves of the tongue can cause contraction of the protrusor muscles to reduce the effect of or prevent OSA.

There are multiple sets of motor points for each of the protrusor muscles on the left side and the right side. Each motor point may innervate one or more muscle fibers of the protrusor muscle. In one example, one lead may be used to stimulate motor points for the protrusor muscles on one side of the tongue, one lead may be used to stimulate motor points for protrusor muscles on both sides of the tongue, or two leads may be used, where each lead stimulates a respective set of motor points for the protrusor muscles on each side. Stimulation of either or both sets of motor points of the tongue can cause contraction of the protrusor muscles to reduce the effect of, or prevent, OSA.

This disclosure describes examples of techniques related to implantation of the one or more leads in the tongue for treatment of OSA. Although the example techniques are described with respect to OSA, the example techniques should not be construed as limited to OSA. Rather, the example techniques described in this disclosure may be applicable to lead implantation for treatment of various conditions, including lead implantation for treatment of conditions where the lead is implanted in a location other than the tongue.

Open surgeries may be performed to implant the one or more leads in a tongue of a patient for treating OSA. However, such open surgeries require dissection of tissue to expose one or more hypoglossal nerves and/or motor points for placement of the one or more leads immediately adjacent to or around the hypoglossal nerves and/or motor points in the tongue of the patient, which is relatively invasive and time-consuming.

In other examples, medical professionals may implant the leads by using a needle to form a path through the tissue of the patient to the hypoglossal nerves of the patient. The one or more leads may then be navigated through the paths to areas adjacent to the hypoglossal nerves and deliver stimulation signals to the hypoglossal nerves. In such examples, the medical professional may first create an initial path using a needle and remove the needle from the patient once the initial path has been created. The medical professional may then advance an introducer attached to a dilator, e.g., over a guidewire or other similar guiding device, to dilate the initial path to an appropriate diameter for the lead and to determine the appropriate orientation for the electrodes of the lead. The medical professional may then remove to the dilator and introducer from the patient and advance an implantable lead sheath into the dilated path. The implantable lead sheath may include an electrically insulative material and may be configured to electrically insulate some portions of the lead while allowing other portions of the lead to deliver stimulation signals to the hypoglossal nerve, e.g., through the one or more electrodes. Finally, the medical professional may insert the lead into an inner lumen of the implantable lead sheath and advance the lead through the inner lumen of the implantable lead sheath to the hypoglossal nerves of the patient to complete the implantation process.

Unlike the examples that require dissecting tissue or examples that require a needle, introducer, guidewire, and other such components, some example techniques utilize a needle configured to percutaneously insert into skin and form a path for inserting a lead, such as without requiring the use of the guidewire or introducer. For instance, the example techniques described in this disclosure may enable a surgeon to implant one or more leads adjacent to or around one or more hypoglossal nerves and/or motor points in the tongue of a patient without dissecting tissue to expose the hypoglossal nerves and/or motor points, which minimize access incision, shorten recovery time for the patient, and reduce risk for misplacement of the leads. In addition, the example techniques described in this disclosure may enable a surgeon to implant the one or more leads adjacent to or around one or more hypoglossal nerves and/or motor points in the tongue of the patient with fewer tools and complete the lead implantation process in a shorter duration.

The example devices, systems, and methods of this disclosure describe example electrical leads with an fixation element. The fixation element may puncture and engage with tissue near the hypoglossal nerve(s) and/or motor points of the patient. The fixation element prevents dislodgement and/or damage to the electrical lead resulting from movement of the tissue around the electrical lead. For example, the fixation element may engage with the tissue at a more distal position than other electrical lead fixation mechanisms, thereby reducing a likelihood of dislodgment of the electrical lead over time. The fixation element may also secure one or more electrodes on the electrical lead at predetermined positions within the tissue and prevent unintended stimulation to other portions of the tissue. In some examples, the fixation element may include an electrode (e.g., a distal electrode) configured to sense electrical signals from and/or deliver electrical stimulation signals to the hypoglossal nerve(s) and/or motor points and may provide a more distal point of stimulation than other electrical leads.

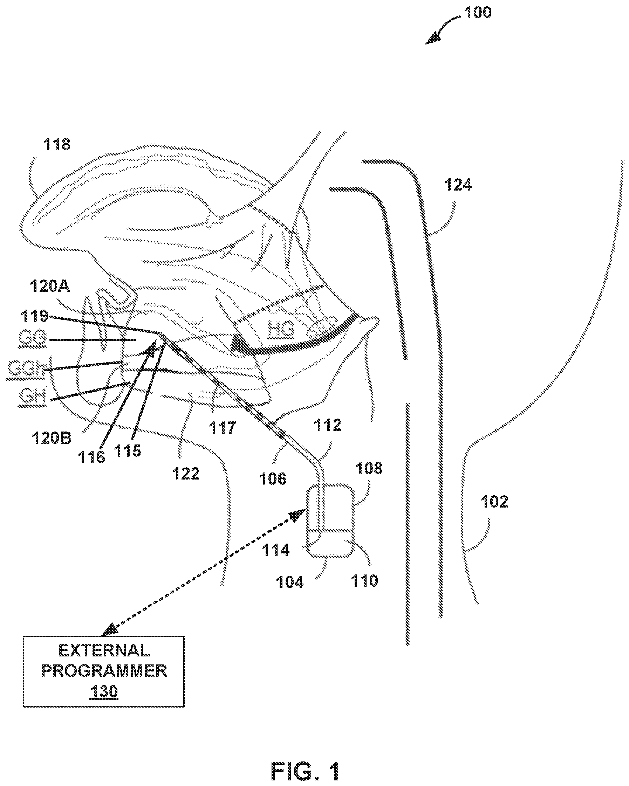

FIG. 1 is a conceptual diagram of a medical system for delivering OSA therapy. In system 100, implantable medical device (IMD) 104 and lead 106 are implanted in patient 102. IMD 104 includes housing 108 enclosing circuitry of IMD 104. In some examples, IMD 104 includes connector assembly 110, which is hermetically sealed to housing 108 and includes one or more connector bores for receiving a proximal end of at least one medical electrical lead 106 (also referred to as "implantable medical lead 106", "lead 106") used for delivering OSA therapy. Although one lead 106 is illustrated in FIG. 1, there may be one or more leads 106 to which IMD 104 is coupled.

Lead 106 may include a flexible, elongated lead body 112 (also referred to as "elongated member 112") extending from lead proximal end 114 to lead distal end 115. As illustrated in FIG. 1, lead 106 includes one or more electrodes 117 that are carried along a lead distal portion adjacent lead distal end 115 and are configured for insertion within the protrusor muscles 120A, 120B, and 122 of tongue 118. As one example, the genioglossus muscle includes oblique compartment 120A and horizontal compartment 120B. In this disclosure, the genioglossus muscle is referred to as protrusor muscle 120. Protrusor muscle 122 is an example of the geniohyoid muscle.

Lead distal end 115 includes one or more electrodes 117 and fixation element 116 (also referred to as "fixation element 116"). Fixation element 116 may be configured to penetrate tissue (e.g., any of protrusor muscles 120A, 120B, and 122 of tongue 118). In some examples, as illustrated in FIG. 1, fixation element 116 is disposed on a distal tip of lead 106. In some examples, fixation element 116 may be disposed along elongated lead body 112 and proximal to at least one of electrodes 117. In some examples, fixation element 116 may include electrically active regions configured to define another electrode (e.g., a distal electrode). The fixation element 116 may be configured to deliver electrical stimulation signals to protrusor muscles 120 and/or 122, e.g., as a part of delivering OSA therapy to patient 102. That is, fixation element 116 may be configured to engage with the tissue of patient 102 to hold lead 106 is in place, and may puncture the tissue of patient 102 to engage with the tissue

Proximal end 114 of lead 106 includes one or more electrical contacts to connect to connector assembly 110. Lead 106 also includes conductors such as coils or wires that connect respective electrodes 117 to respective electrical contacts at proximal end 114 of lead 20. One or more conductors such as coils or wires may also connect fixation element 116 to respective electrical contacts.

**During** implantation of system 100, a clinician may insert a needle within the protrusor muscles 120A, 120B, and 122 of tongue 118. The clinician may then deliver test stimulation signals to protrusor muscles 120 and/or 122 via the needle and sense, via system 100, electrical signals (e.g., evoked electrical signals) from the tissue of patient 102 in response to the test stimulation signals. The clinician may determine, based on the sensed electrical signals, if needle is placed in a proper location within protrusor muscles 120 and/or 122 to deliver stimulation to hypoglossal nerve(s) and/or motor points of patient 102. The clinician may iteratively re-position the needle and transmit test stimulation signals until the clinician determines that the needle is properly placed within protrusor muscles 120 and/or 122.

The clinician may then retract the needle and advance an introducer into a path to protrusor muscles 120 and/or 122 created by the needle. The use of an introducer may be optional, but for ease of description, the use of introducer is described. The clinician may then advance lead 106 into protrusor muscles 120 and/or 122 via an introducer lumen defined by the introducer or directly through lumen of needle if introducer is not used. The clinician may deliver test stimulation signals to protrusor muscles 120 and/or 122 via one or more electrodes 117 and/or a distal electrode defined by at least a portion of fixation element 116 and sense, via system 100, electrical signals (e.g., evoked electrical signals) from the tissue of patient 102 in response to the test stimulation signals. The clinician may determine, based on the sensed electrical signals, if electrodes 117 and/or the distal electrode are placed in a proper location within protrusor muscles 120 and/or 122 to deliver stimulation to hypoglossal nerve(s) and/or motor points of patient 102. The clinician may iteratively reposition lead 106 and transmit test stimulation signals until the clinician determines that electrodes 117 and/or the distal electrode is properly placed within protrusor muscles 120 and/or 122.

Once the clinician determines that lead 106 is properly placed within protrusor muscles 120 and/or 122, the clinician may secure lead 106 within protrusor muscles 120 and/or 122 via fixation element 116. The clinician may secure lead 106 to tissue of protrusor muscles 120 and/or 122 by applying a torque to fixation element 116 to rotate fixation element 116 about a longitudinal axis and to advance fixation element 116 into and engage with tissue of patient 102. The clinician may apply the torque to fixation element 116 via a rotation member configured to engage with lead 106 (e.g., a locking sheath disposed over lead 106, an elongated stylet disposed within a lead lumen, or the like). Once fixation element 116 is securely engaged to protrusor muscles 120 and/or 122, the clinician may retract introducer proximally from the path and complete the implantation process. In some examples, where the clinician may need to remove lead 106 from protrusor muscles 120 and/or 122, the clinician may apply a torque to fixation element 116 in an opposite direction to retract fixation element 116 from tissue of patient 102 and release lead 106 from protrusor muscles 120 and/or 122.

Engaging with protrusor muscles 120 and/or 122 is provided as one example, and the techniques should not be considered limited. Fixation element 116 may engage with other tissue in addition to or instead of protrusor muscles 120 and/or 122. For example purposes, fixation element 116 is described as engaging with protrusor muscles 120 and/or 122.

While protrusor muscles 120 and 122 are described, the example techniques described in this disclosure are not limited to stimulating near protrusor muscles 120 and 122. Also, FIG. 1 illustrates one set of protrusor muscles 120 and 122 (e.g., on a first side of tongue 118). The other side of tongue 118 also includes protrusor muscles. For instance, a left side of tongue 118 includes a first set of protrusor muscles 120 and 122, and a right side of tongue 118 includes a second set of protrusor muscles.

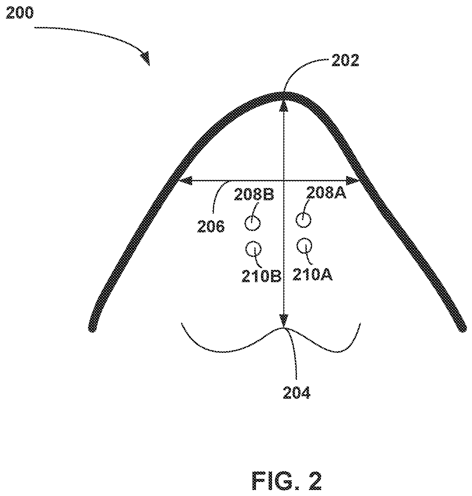

In some examples, the clinician may implant one or more leads 106 such that one or more electrodes 117 are implanted within soft tissue, such as musculature, proximate to medial branches of one or both hypoglossal nerves. In some examples, one or more electrodes 117 may be approximately 5 mm (e.g., 2 mm to 8 mm) from a major trunk of the hypoglossal nerve. In some examples, one or more electrodes 117 may be placed in an area of protrusor muscles 120 and 122 that include motor points, where each nerve axon terminates in the muscle (also called the neuro-muscular junction). The motor points are not at one location but spread out in the protrusor muscles. Leads 106 may be implanted such that one or more electrodes 117 may be generally in the area of the motor points (e.g., such that the motor points are within 1 to 10 mm from one or more electrodes 117). Examples of motor points for protrusor muscles 120 and 46 are illustrated in more detail with respect to FIG. 2.

Tongue 118 includes a distal end (e.g., tip of tongue 118), and electrodes 117 may be implanted proximate to a root of tongue 118. The surgeon may implant one or more leads 106 such that one or more electrodes 117 and/or distal electrode 119 are implanted proximate to the root of tongue 118, as illustrated in FIG. 1. For example, the location for stimulation for the genioglossus muscle 120 may be approximately 30 mm (e.g., 25 mm to 35 mm) from the symphysis of the jaw (e.g., where the genioglossus and hypoglossal muscles insert). The location for stimulation for the geniohyoid muscle 122 may be approximately 40 mm (e.g., 35 mm to 45 mm) from the symphysis. For both the genioglossus muscle 120 and the geniohyoid muscle 122, the location for stimulation may be approximately 11 mm (e.g., 7 mm to 15 mm) lateral to the midline on both the right and left sides of tongue 118 for stimulating respective hypoglossal nerves. In some examples, rather than stimulating hypoglossal nerves, the examples described in this disclosure may be configured for stimulating the motor points, as described in more detail with respect to FIG. 2. Stimulating the motor points may result in indirect activation of the hypoglossal nerve but may generally be stimulating at a different location than direct stimulation to the hypoglossal nerve. As a result, in some examples, simulation of one or more motor points may result in more precise activation of muscle fibers than may be possible with stimulation of the hypoglossal nerve itself.

One or more electrodes 117 of lead 106 may be ring electrodes, segmented electrodes, partial ring electrodes, or any suitable electrode configuration. Ring electrodes extend 360 degrees around the circumference of lead body 112 of lead 106. Segmented and partial ring electrodes each extend along an are less than 360 degrees (e.g., 90-120 degrees) around the outer circumference of lead body 112 of lead 106. In this manner, multiple segmented electrodes may be disposed around the perimeter of lead 106 at the same axial position of the lead. In some examples, segmented electrodes may be useful for targeting different fibers of the same or different nerves at respective circumferential positions with respect to the lead to generate different physiological effects (e.g., therapeutic effects), permitting stimulation to be oriented directionally. In some examples, lead 106 may be, at least in part, paddle-shaped (e.g., a "paddle" lead), and may include an array of electrodes arranged as contacts or pads on a common surface, which may or may not be substantially flat and planar.

As described above, in some examples, electrodes 117 and/or distal electrode 119 of lead 106 are disposed within the musculature of tongue 118. Accordingly, one or more electrodes 117 and/or distal electrode 119 of lead 106 may be "intramuscular electrodes." Intramuscular electrodes may be different than other electrodes that are placed on or along a nerve trunk or branch, such as a cuff electrode, used to directly stimulate the nerve trunk or branch. The example techniques described in this disclosure are not limited to intramuscular electrodes and may be extendable to electrodes placed closer to a nerve trunk or branch of the hypoglossal nerve(s). Also, in some examples, one or more electrodes 117 of lead 106 may be implanted in connective tissue or other soft tissue proximate to the hypoglossal nerve.

In some examples, the needle may be configured for advancement through the soft tissue, which may include the protrusor muscle tissue, to form a path configured to place lead 106 electrodes 117 of lead 106, and/or fixation element 116 in proximity to the hypoglossal nerve(s) that innervate protrusor muscles 120 and/or 122 and/or motor points that connect axons of hypoglossal nerve(s) to respective muscle fibers of protrusor muscles 120 and/or 122. In some examples, the needle is used for vascular implantation. In such examples, the needle may include a hemostasis valve positioned within an attachment member disposed on a proximal portion of the needle assembly. The hemostasis valve may prevent transfer of blood or other bodily fluids into the needle assembly.

As described above, electrical stimulation therapy generated by IMD 104 and delivered via one or more electrodes 117 and/or the distal electrode 119 may activate protrusor muscles 120 and 122 to move tongue 118 forward, for instance, to promote a reduction in obstruction or narrowing of the upper airway 124 during sleep. As used herein, the term "activated" with regard to the electrical stimulation of protrusor muscles 120 and 122 refers to electrical stimulation that causes depolarization or an action potential of the cells of the nerve (e.g., hypoglossal nerve(s)) or stimulation at the neuro-muscular junction between the nerve and the protrusor muscles (e.g., at the motor points) innervating protrusor muscles 120 and 122 and motor points and subsequent depolarization and mechanical contraction of the protrusor muscle cells of protrusor muscles 120 and 122. In some examples, protrusor muscles 120 and 122 may be activated directly by the electrical stimulation therapy.

Protrusor muscles 120 and/or 122, on a first side of tongue 118 (e.g., the left or right side of tongue 118), may be activated by a medial branch of a first hypoglossal nerve, and the protrusor muscles, on a second side of tongue 118 (e.g., the other of the left or right side of tongue 118), may be activated by a medial branch of a second hypoglossal nerve. The medial branch of a hypoglossal nerve may also be referred to as the XIIth cranial nerve. The hyoglossus and styloglossus muscles (not shown in FIG. 1), which cause retraction and elevation of tongue 118, are activated by a lateral branch of the hypoglossal nerve.

One or more electrodes 117 and/or distal electrode 119 may be used to deliver bilateral or unilateral stimulation to protrusor muscles 120 and 122 via the medial branch of the hypoglossal nerve or branches of the hypoglossal nerve (e.g., such as at the motor point where a terminal branch of the hypoglossal nerve interfaces with respective muscle fibers of protrusor muscles 120 and/or 122). For example, one or more electrodes 117 and/or distal electrode 119 may be coupled to output circuitry of IMD 104 to enable delivery of electrical stimulation pulses in a manner that selectively activates the right and left protrusor muscles (e.g., in a periodic, cyclical, or alternating pattern) to avoid muscle fatigue while maintaining upper airway patency. Additionally, or alternatively, IMD 104 may deliver electrical stimulation to selectively activate protrusor muscles 120 and/or 122 or portions of protrusor muscles 120 and/or 122 during unilateral stimulation of the left or right protrusor muscles.

In some examples, one lead 106 may be implanted such that one or more of electrodes 117 and/or distal electrode 119 may deliver electrical stimulation to stimulate the left hypoglossal nerve or motor points of protrusor muscles on the left side of tongue, and therefore cause the left protrusor muscles to activate. In such examples, the electrical stimulation from one or more electrodes 117 and/or distal electrode 119 may not be of sufficient amplitude to stimulate the right hypoglossal nerve or motor points of protrusor muscles on the right side of tongue and cause the right protrusor muscles to activate. In some examples, one lead 106 may be implanted such that one or more of electrodes 117 and/or distal electrode 119 delivers electrical stimulation to stimulate the right hypoglossal nerve or motor points of protrusor muscles on the right side of tongue, and therefore cause the right protrusor muscles to activate. In such examples, the electrical stimulation from one or more electrodes 117 and/or distal electrode 119 may not be of sufficient amplitude to stimulate the left hypoglossal nerve or motor points of protrusor muscles on the left side of tongue and cause the left protrusor muscles to activate. Accordingly, in some examples, two leads like lead 106 may be implanted to stimulate each of the left and right hypoglossal nerves and/or motor points of respective protrusor muscles on the left and right side of tongue 118.

In some examples, one lead 106 may be implanted substantially in the middle (e.g., center) of tongue 118. In such examples, one or more electrodes 117 and/or distal electrode 119 may deliver electrical stimulation to both hypoglossal nerves or motor points of both muscles on both sides of tongue 118, causing both hypoglossal nerves or motor points to activate respective left and right protrusor muscles. It may be possible to utilize current steering and field shaping techniques such that one or more electrodes 117 and/or distal electrode 119 deliver first electrical stimulation that stimulates the left hypoglossal nerve or motor points of protrusor muscles on the left side of tongue 118 with little to no stimulation of the right hypoglossal nerve or motor points of protrusor muscles on the right side of tongue 118, and then one or more electrodes 117 and/or distal electrode 119 deliver second electrical stimulation that stimulates the right hypoglossal nerve or motor points of protrusor muscles on the right side of tongue with little to no stimulation of the left hypoglossal nerve or motor points of protrusor muscles on the left side of tongue. In examples where two leads like lead 106 are utilized, each lead may alternate delivery of stimulation to respective hypoglossal nerves or motor points. In this way, IMD 104 may stimulate one hypoglossal nerve or one set of motor points and then the other hypoglossal nerve or another set of motor points, which can reduce muscle fatigue.

For instance, continuous stimulation may cause protrusor muscles to be continuously in an advanced state. This continuous contraction may cause protrusor muscles 120 and/or 122 to fatigue. In such cases, due to fatigue, the stimulation may not cause protrusor muscles 120 and/or 122 to maintain an advanced state (or higher intensity of the electrical stimulation may be needed to cause protrusor muscles 120 and/or 122 to remain in the advanced state). By stimulating one set of protrusor muscles (e.g., left or right), a second set (e.g., other of left or right) of protrusor muscles can be at rest. Stimulation may then alternate to stimulate the protrusor muscles that were at rest and thereby maintain protrusion of tongue 118 while permitting the protrusor muscles 120 and/or 122 that were previously activated to rest. Hence, by cycling between alternate stimulation of the left and right protrusor muscles, tongue 118 can remain in the advanced state, while one of the first or second set of protrusor muscles is at rest.

In some examples, one lead 106 may be implanted laterally or diagonally across tongue 118 such that some of electrodes 117 and/or distal electrode 119 on lead 106 can be used to stimulate the left hypoglossal nerve and/or motor points of the protrusor muscles on the left side of tongue 118 and some of electrodes 117 and/or distal electrode 119 on the same lead 106 can be used to stimulate the right hypoglossal nerve and/or motor points of the protrusor muscles on the right side of tongue 118. In such examples, IMD 104 may selectively deliver electrical stimulation to a first hypoglossal nerve and/or first motor points of the protrusor muscles on a first side of tongue 118 via a first set of one or more electrodes 117 and/or distal electrode 119, and then deliver electrical stimulation to a second hypoglossal nerve and/or second set of motor points of the protrusor muscles on a second side of tongue 118 via a second set of one or more electrodes 117 and/or distal electrode 119. This may be another way in which to reduce muscle fatigue.

Lead proximal end 114 includes a connector (not shown in FIG. 1) that may be coupled to connector assembly 110 of IMD 104 to provide electrical connection between circuitry enclosed by the housing 108 of IMD 104. Lead body 112 encloses electrical conductors extending from each of one or more electrodes 117 and/or distal electrode 119 to the proximal connector at proximal end 114 to provide electrical connection between output circuitry of IMD 104 and the electrodes 117.

There may be various ways in which lead 106 is implanted in patient 102. A clinician may insert the needle through the lower part of the jaw and in tongue 118 starting from the back of tongue 118. The clinician may insert the needle until a distal tip of the needle reaches a point at or adjacent to the root of tongue 118, angling the needle to extend proximate to the hypoglossal nerve (e.g., left or right hypoglossal nerve). In some examples, the needle may include one or more electrically conductive areas (e.g., one or more electrodes) at the distal end, and the clinician may cause the one or more electrically conductive areas of the needle to output electrical stimulation (e.g., in the form of controlled current pulses or controlled voltage pulses), which in turn causes a physiological response such as activation of protrusor muscles 120 and/or 122 and advancement of tongue 118. In some examples the one or more electrodes may be disposed on an outer surface of the needle. The clinician may adjust the location of the needle based on the physiological response to determine a location in tongue 118 that provides effective treatment.

In some examples, once the needle is implanted within tongue 118, the clinician may retract needle from within tongue 118, advance an introducer into tongue 118 via a path formed by the needle, and advance lead 106 through the introducer lumen of the introducer. The clinician may determine lead 106 is at a desired position within tongue 118, secure lead 106 within tongue 118 via fixation element 116, and retract the introducer from within tongue 118 once lead 106 is secured within tongue 118.

IMD 104 may output stimulation signals through electrodes 117 and/or distal electrode 119 to stimulate the hypoglossal nerve and/or one or more motor points of the protrusor muscle within tongue 118. If further refinement is needed to determine the lead placement for lead 106, the clinician may adjust the location of needle and/or lead 106 within patient 102 in response to one or more electrical signals detected by electrodes 117, distal electrode 119 of fixation element 116, and/or electrodes on the needle. During implantation and testing of lead 106, IMD 104 may not yet be implanted within body of patient 102. After completing implantation and testing of lead 106, the clinician may implant IMD 104 within patient 102 (e.g., in the neck of patient 102, in the torso of patient 102, or the like) to complete the implantation of system 100. In some examples, lead 106 may be connected to another computing device and/or system (e.g., an external programming device) and the another computing device and/or system may output the stimulation signals for purposes of delivering the lead placement for lead 106.

As an example, some other techniques of implanting lead 106 include using a needle to percutaneously insert into the skin. A clinician places a guidewire through the lumen of needle, then removes the needle. The guidewire remains in place in the tissue in original location as the needle inside patient 102. The clinician places an introducer sheath, possibly with a dilator, over the guidewire. The clinician removes the guidewire, and places lead 106 into the introducer sheath. The clinician engages fixation element 116 with the tissue of patient 102 Once fixation element 116 is engaged, the clinician then removes the introducer sheath leaving lead 106 in place.

Fixation element 116 described in this disclosure may provide one or more benefits over fixation elements/mechanisms of other implantable medical leads. Fixation element 116 may provide improved engagement with protrusor muscles 120 and/or 122 of patient 102 and reduce a likelihood of dislodgement of lead 106. Fixation element 116 may also affix lead 106 at a position deeper within protrusor muscles 120 and/or 122 which may increase the likelihood of affixing lead 106 to muscle tissue, thereby preventing dislodgement of lead 106. In some examples, fixation element 116 described herein may also reduce an outer diameter of lead body 112 and may facilitate repositioning of lead 106 within the path formed by the needle within protrusor muscles 120 and/or 122.

In some examples, fixation element 116 includes an elongated member defining a helical or spiral shape. In other examples, the elongated member may define another shape. A distal tip of fixation element 116 may puncture tissue of patient 102, e.g., at a distal end of the path formed by the needle. Fixation element 116 is configured to at least partially advance into the puncture created by the distal tip. Once advanced into the puncture, portions of fixation element 116 may engage with surround tissue to fixation element 116 and lead 106 within the tissue. The clinician may advance fixation element 116 into the puncture in the tissue by applying a torque to fixation element 116 Once secured, fixation element 116 may resist linear forces along the longitudinal axis of fixation element 116 and/or lead 106. Lead 106 may include features (e.g., protrusions) configured to prevent unintended rotation of fixation element 116 within the tissue of patient 102.

At least a portion (e.g., a distal portion) of fixation element 116 defines a distal electrode 119 configured to sensed electrical signals from and/or deliver electrical stimulation signals to the tissue surrounding distal electrode 119. In some examples, distal electrode 119 may be electrically connected to IMD 104 via an electrical conductor disposed at proximal end 114 of lead 106. Distal electrode 119 of fixation element 116 may sense the electrical signals and/or deliver electrical stimulation signals in a same manner as one or more of electrodes 117, as described above.

In any of the manners described above, the surgeon may implant one lead 106. In examples where two or more leads are implanted, the surgeon may perform steps similar to those described above.

The above describes some example techniques for lead placement, and the examples described in this disclosure should not be considered limited to such examples of lead placement. Moreover, in some examples, the surgeon may use imaging techniques, such as fluoroscopy, during implantation to verify proper placement of lead 106, the needle, and/or the introducer.

FIG. 1 illustrates the location of IMD 104 as being within or proximate to the neck of patient 102. However, IMD 104 may be implanted in various other locations. As one example, the surgeon may implant IMD 104 in the left or right pectoral region. For instance, the surgeon may plan on implanting IMD 104 in the left pectoral region unless another medical device is already implanted in the left pectoral region. If another medical device is already implanted in the left pectoral region, the surgeon may then implant IMD 104 in the right pectoral region. There may include other locations where the surgeon may implant IMD 104, such as the back of patient 102. The example techniques are not limited to any particular implant location of IMD 104.

In accordance with one or more examples described in this disclosure, system 100 is an implant system for utilizing lead 106 in tongue 118 for treatment of OSA. In some examples, system 100 may be configured such that substantial dissection is not required to expose one or more hypoglossal nerves and/or one or more motor points of the protrusor muscle within tongue 118 for placement of the lead. In some examples, system 100 may be configured such that a surgeon may implant the needle and lead 106 within patient 102 using a relatively smaller number of devices (e.g., without the use of an introducer sheath, guide members (e.g., a guidewire), a dilator, and the like). This disclosure describes examples of system 100 configured for placement of lead 106 in a way that minimizes access incisions for placement of lead 106.

In some situations, it may be desirable to include multiple electrodes on lead 106 to achieve desired physiological effects (e.g., therapeutic effects). For example, to achieve the desired effect, multiple electrodes may be used to target different fibers of the same nerve (e.g., target one or more motor points of the protrusor muscle within tongue 118). In such cases, determining the locations of the different fibers or motor points one at a time is time-consuming and may cause nerve injury. In some examples, system 100 may enable a surgeon to identify the locations of different fibers or motor points of the protrusor muscles in such a manner to shorten the surgical time and reduce the risk of nerve injury.

As described above, system 100 is an implant system for implanting lead 106 adjacent to or around one or more hypoglossal nerves and/or motor points without open surgery, so that lead 106 may be implanted to stimulate the nerves with minimal impact to patient 102. There may be certain unique challenges associated with implanting lead 106 adjacent to or around the hypoglossal nerves and/or the one or more motor points of the protrusor muscle (e.g., protrusor muscles 120 and/or 122) in tongue 118 without open surgery. As one example, without performing an open surgery to expose the hypoglossal nerves and/or motor points, there are difficulties with localizing and accessing the hypoglossal nerves and/or motor points.

To identify the location of a hypoglossal nerve and/or a motor point without performing an open surgery, system 100 may include the needle for creating an opening in tongue 118 of the patient for implantation of lead 106 and a medical device for delivering stimulation signals through lead 106 and the needle to tongue 118 of patient 102 to stimulate the hypoglossal nerve and/or the motor point. The same medical device or possibly another medical device may further receive electrical signals from lead 106, where the electrical signals (e.g., EMG signals) are generated from a muscle movement in response to the stimulation signals. As illustrated in FIG. 1, the medical device may be an implantable medical device (e.g., IMD 104) implanted near the neck of patient 102. Hence, IMD 104 may be utilized for chronic (i.e., long-term) treatment of OSA. However, in some examples, during the implantation of lead 106 or determining location for implanting lead 106, a trial stimulator (e.g., external medical device) may be used to deliver stimulation to the needle or through lead 106 when lead 106 is within the needle. Accordingly, the medical device may be an external medical device coupled to lead 106 and/or the needle for delivering and/or detecting stimulation signals. It should be noted that IMD 104 may also be used as a trial stimulator, and the techniques are not limited to an external medical device.

System 100 includes an needle that has an elongated body (e.g., the needle body). In some examples, the needle may include one or more electrodes positioned on the outer surface of the elongated body. The one or more electrodes positioned on the outer surface of the elongated body are different than one or more electrodes 117, where electrodes 117 are on lead 106. The one or more electrodes may be configured to deliver electrical stimulation signals to tongue 118 of patient 102 and/or detect electrical signals from tongue 118. Electrodes configured to deliver electrical stimulation may also be referred herein as "stimulation electrodes" and electrodes configured to detect electrical signals may also be referred herein as "sensing electrodes."

System 100, along with the needle and lead 106, also includes a medical device for delivering stimulation signals via the needle through one or more stimulation electrodes on the needle to tongue 118 of patient 102 to stimulate a hypoglossal nerve and/or a motor point in tongue 118 of patient 102 as part of the implantation procedure. The medical device may also receive one or more electrical signals detected by the needle through one or more sensing electrodes and output information indicative of the one or more electrical signals. For example, the medical device may receive an EMG signal that measures an electrical current generated from a muscle contraction in response to the stimulation signal.

For instance, a clinician may insert the needle in tongue 118 of patient 102 such that one or more electrodes on the needle are pushed through tissue near a chin of patient 102 and through tongue 118 proximate to the hypoglossal nerve and/or the motor point of a protrusor muscle (e.g., protrusor muscles 120 and/or 122) within tongue 118. After inserting the needle, the clinician may control the medical device to deliver a stimulation signal via the needle through the one or more stimulation signals to tongue 118 of patient 102 to stimulate the hypoglossal nerve and/or the motor point in tongue 118 of patient 102. The clinician may also control the medical device (same or different medical device) to receive electrical signals detected by the needle through one or more sensing electrodes and output information indicative of the one or more electrical signals on a display device. The clinician may then determine a target treatment site based on the output information indicative of the one or more electrical signals.

FIG. 2 is a conceptual diagram illustrating example locations of motor points where stimulation for OSA therapy may be delivered. FIG. 2 illustrates jaw 200 of patient 102, where patient 102 is in a supine position and jaw 200 of patient 102 is viewed from an inferior location of patient 102. For instance, FIG. 2 illustrates symphysis 202 and hyoid bone 204. In the example illustrated in FIG. 2, the line interconnecting symphysis 202 and hyoid bone 204 may be considered as a y-axis along the midline of tongue 118. FIG. 2 also illustrates intergonial distance 206 between the two gonia of patient 102, where the gonia is a point on each side of the lower jaw 200 at the mandibular angle. Intergonial distance 206 may be along the x-axis of tongue 118. While FIG. 2 illustrates delivery of electrical stimulation signals at the illustrated example locations of motor points, the example systems described herein may deliver stimulation for OSA therapy to other motor points of patient 102, only to hypoglossal nerve(s) of patient 102, or to any other location in tongue 118 of patient 102.

FIG. 2 illustrates motor points 208A and 208B and motor points 210A and 210B. Motor points 208A may be motor points for the right genioglossus muscle, and motor points 208B may be motor points for the left genioglossus muscle. Motor points 210A may be motor points for the right geniohyoid muscle, and motor points 210B may be motor points for the left geniohyoid muscle. Motor points 208A and 208B and motor points 210A and 210B may genericize the motor points for each muscle for purposes of illustration. There may be additional motor points and/or motor points at different locations for each muscle.

In one or more examples, the needle, lead 106, and/or one or more electrodes 117 may be implanted proximate to motor points 208A, 208B, 210A, or 210B for stimulating at motor points 208A, 208B, 210A, and/or 210B. For instance, in examples where two leads are implanted, a first lead and its electrodes may be implanted proximate to motor points 208A and/or 210A and a second lead and its electrodes may be implanted proximate to motor points 208B and/or 210B. In one or more examples, electrodes 117 may be approximately 1 mm to 10 mm from respective motor points 208A, 208B, 210A, or 210B.

A hypoglossal nerve (e.g., on the left or right side of tongue 118) initially is a trunk of nerves fibers called axons. The axons of the hypoglossal nerve branch out. For example, the trunk of hypoglossal nerve includes multiple sets of axons including a first set of axons, and the first set of axons branch out from the trunk of the hypoglossal nerve. The first set of axons include multiple groups of axons including a first group of axons, and the first group of axons branch out from the first set of axons, and so forth. The locations where the branched-out axons interface with respective muscle fibers of protrusor muscles 120 and/or 122 (e.g., genioglossus and/or geniohyoid muscle) are referred to as motor points.

For instance, a branch of the hypoglossal nerve that interfaces (e.g., connects at the neuro-muscular junction) with the muscle fiber is referred to as a terminal branch, and the end of the terminal branch is a motor point. The length of a terminal branch may be approximately 10 mm from the hypoglossal nerve to the genioglossal or geniohyoid muscles. In some examples, there may be approximately an average of 1.5 terminal branches with a standard deviation of ± 0.7 for the right geniohyoid muscle, an average of 4.8 terminal branches with a standard deviation of ± 1.4 for the right genioglossus muscle, an average of 2.0 terminal branches with a standard deviation of ± 0.9 for the left geniohyoid muscle, and an average of 5.1 terminal branches with a standard deviation of ± 1.9 for the left genioglossus muscle.

There may be possible advantages with stimulating at motor points 208A, 208B, 210A, or 210B, as compared to some other techniques. For instance, some techniques utilize cuff electrodes or stimulate at the hypoglossal nerve. Due to the different bifurcation patterns, placing a cuff electrode around the hypoglossal nerve, or generally attaching an electrode to the hypoglossal nerve can be challenging. Also, where cuff electrodes or electrodes that attach to the hypoglossal nerve are used, implanting electrodes around or at each of the hypoglossal nerves requires multiple surgical entry points to attached to both hypoglossal nerves. Moreover, utilizing cuff electrodes or electrodes that attach to the hypoglossal nerves can possibly negatively impact the nerve by tugging, stretching, or otherwise causing irritation. Accordingly, utilizing lead 106 and electrodes 117 that are implanted proximate to the motor points may be beneficial (e.g., less surgery to implant and less impact on the nerve) as compared to techniques where cuff electrodes or electrodes implanted on the hypoglossal nerve are utilized.

Furthermore, stimulating at motor points 208A, 208B, 210A, and/or 210B, such as at the bifurcation point of a motor neuron that attach to muscle fibers, may provide advantages such as for better control of muscle movement. Because motor points 208A, 208B, 210A, and 210B are spatially distributed, by stimulating motor points 208A, 208B, 210A, and/or 210B, the amount of the genioglossus and geniohyoid muscle that is being stimulated can be controlled. Also, stimulating at motor points 208A, 208B, 210A, and/or 210B may allow for more gentle muscle activation. For instance, when stimulation is provided near the trunk of the hypoglossal nerve, even stimulation signal with relatively small amplitude can cause the genioglossus and/or geniohyoid muscle to fully protrude (e.g., there is high loop gain where small stimulation amplitudes cause large muscle protrusion). Fine tuning of how much to protrude the genioglossus and/or geniohyoid muscle may not be available when stimulating at a trunk of the hypoglossal nerve. However, there may be lower loop gain stimulating at motor points 208A, 208B, 210A, and/or 210B. For instance, a stimulation signal having a lower amplitude may move cause the genioglossus and/or geniohyoid muscle to protrude a small amount, and a stimulation signal having a higher amplitude may move cause the genioglossus and/or geniohyoid muscle to protrude a higher amount when stimulating at motor points 208A, 208B, 210A and/or 210B.

The following are example locations of motor points 208A, 208B, 210A, and 210B relative to the midline (x-axis), posterior symphysis 202 (y-axis), and depth (z-axis), where the depth is from the plane formed by the inferior border of symphysis 202 and anterior border of hyoid bone 204.

Motor points 208A may be for the right genioglossus muscle and may be located at 13.48 mm ± 3.59 mm from the x-axis, 31.01 mm ± 6.96 mm from the y-axis, and 22.58 mm ± 3.74 mm from the z-axis. Motor points 210A may be for the right geniohyoid muscle and may be located at 11.74 mm ± 3.05 mm from the x-axis, 41.81 mm ± 6.44 mm from the y-axis, and 16.29 mm ± 3.40 mm from the z-axis. Motor points 208B may be for the left genioglossus muscle and may be located at 9.96 mm ± 2.24 mm from the x-axis, 29.62 mm + 9.25 mm from the y-axis, and 21.11 mm + 4.10 mm from the z-axis. Motor points 210B may be for the left geniohyoid muscle and may be located at 11.45 mm ± 1.65 mm from the x-axis, 39.63 mm ± 8.03 mm from the y-axis, and 15.09 mm + 2.41 mm from the z-axis.

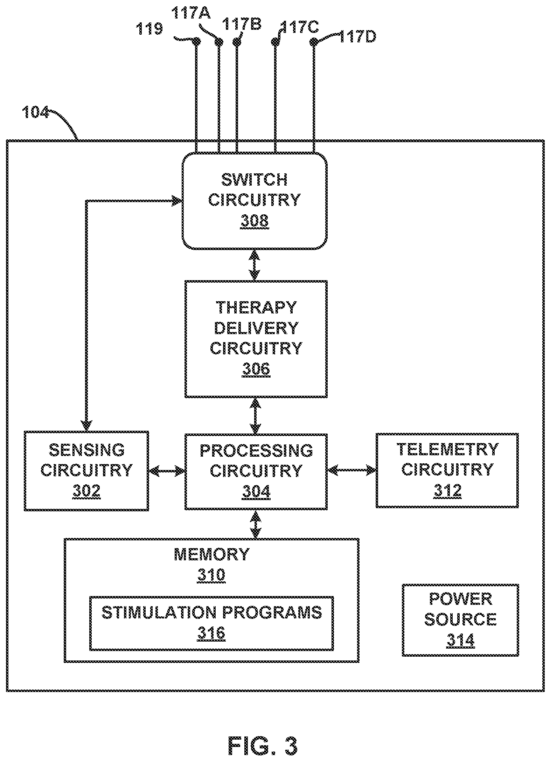

FIG. 3 is block diagram illustrating example configurations of implantable medical devices (IMDs) which may be utilized in the system of FIG. 1. As shown in FIG. 3, IMD 104 includes sensing circuitry 302, processing circuitry 304, therapy delivery circuitry 306, switch circuitry 308, memory 310, telemetry circuitry 312, and power source 314. IMD 104 may include a greater or fewer number of components. For example, in some examples, such as examples in which IMD 104 deliver the electrical stimulation in an open-loop manner, IMD 104 may not include sensing circuitry 302.

**Switch** circuitry 308 may be configured to, in response to instructions from processing circuitry 304, switch the coupling of electrodes 117 and/or distal electrode 119 of fixation element 116 (not pictured) between sensing circuitry 302 and therapy delivery circuitry 306. In examples where sensing circuitry 302 is not used, switch circuitry 308 may not be needed. However, even in examples where sensing circuitry 302 is not used, IMD 104 may include switch circuitry 308, e.g., to disconnect electrodes 117 and/or distal electrode 119 from therapy delivery circuitry 306.

In some examples, therapy delivery circuitry 306 may include a plurality of regulated current sources or sinks, with each current source or sink coupled to one of electrodes 117. In such examples, therapy delivery circuitry 306 may control each current source or sink and switching between electrodes 117 may not be necessary for therapy delivery since each one of electrodes 117 is individually controllable.

Although not shown in FIG. 3, in some examples, IMD 104 may include one or more sensors configured to sense posture or position of patient 102. For example, IMD 104 may include accelerometer to determine if patient 102 is lying down. Another example of the one or more sensors is a motion sensor, and movement sensed by the motion sensor may indicate if patient 102 is having restless sleep, which may be indicative of the onset of OSA. Additional examples of the sensors include acoustical sensors or a microphone for detecting vibrations in upper airway 124. Vibrations in upper airway 124 may be indicative of the onset of OSA. In some examples, processing circuitry 304 may control delivery of therapy based on information received from the one or more sensors, such as delivery of therapy after sensing an onset of OSA.

In some examples, electrodes 117 may be configured to sense electromyogram (EMG) signals. Sensing circuitry 302 may be switchably coupled to electrodes 117 via switch circuitry 308 to be used as EMG sensing electrodes with electrodes 117 are not being used for stimulation. EMG signals may be used by processing circuitry 304 to detect sleep state and/or low tonal state of protrusor muscles 120 and/or 122 for use in delivering electrical stimulation. In some examples, rather than using electrodes 117 or in addition to using electrodes 117, there may be other electrodes or sensors used to sense EMG signals.

In general, IMD 104 may comprise any suitable arrangement of hardware, alone or in combination with software and/or firmware, to perform the techniques attributed to IMD 104 and processing circuitry 304, therapy delivery circuitry 306, and telemetry circuitry 312 of IMD 104. In various examples, IMD 104 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components.

The various units of IMD 104 may be implemented as fixed-function circuits, programmable circuits, or a combination thereof. Fixed-function circuits refer to circuits that provide particular functionality and are preset on the operations that can be performed. Programmable circuits refer to circuits that can be programmed to perform various tasks, and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. In some examples, one or more of the units may be distinct circuit blocks (fixed-function or programmable), and in some examples, one or more of the units may be integrated circuits.

IMD 104 may include arithmetic logic units (ALUs), elementary function units (EFUs), digital circuits, analog circuits, and/or programmable cores, formed from programmable circuits. In examples where the operations of IMD 104 are performed using software executed by the programmable circuits, memory 310 may store the instructions (e.g., object code) of the software that processing circuitry 304 receives and executes, or another memory within IMD 104 (not shown) may store such instructions.

IMD 104 also, in various examples, may include a memory 310, such as random access memory (RAM), read only memory (ROM), programmable read only memory (PROM), erasable programmable read only memory (EPROM), electronically erasable programmable read only memory (EEPROM), flash memory, comprising executable instructions for causing the one or more processors to perform the actions attributed to them. Moreover, although sensing circuitry 302, processing circuitry 304, therapy delivery circuitry 306, switch circuitry 308, and telemetry circuitry 312 are described as separate circuitry, in some examples, sensing circuitry 302, processing circuitry 304, therapy delivery circuitry 306, switch circuitry 308, and telemetry circuitry 306 are functionally integrated. In some examples, sensing circuitry 302, processing circuitry 304, therapy delivery circuitry 306, switch circuitry 308, and telemetry circuitry 312 correspond to individual hardware units, such as ASICs, DSPs, FPGAs, or other hardware units.

Memory 310 stores stimulation programs 316 (also called "therapy programs 316") that specify stimulation parameter values for the electrical stimulation provided by IMD 104. Memory 310 may also store instructions for execution by processing circuitry 304, in addition to stimulation programs 316. Information related to sensed parameters of patient 102 (e.g., from sensing circuitry 302 or the one or more sensors of IMD 104) may be recorded for long-term storage and retrieval by a user, and/or used by processing circuitry 304 for adjustment of stimulation parameters (e.g., amplitude, pulse width, and pulse rate). In some examples, memory 310 includes separate memories for storing instructions, electrical signal information, and stimulation programs 316. In some examples, processing circuitry 304 may select new stimulation parameters for a stimulation program 316 or new stimulation program from stimulation programs 316 to use in the delivery of the electrical stimulation based on patient input and/or monitored physiological states after termination of the electrical stimulation.

Generally, therapy delivery circuitry 306 generates and delivers electrical stimulation under the control of processing circuitry 304. In some examples, processing circuitry 304 controls therapy delivery circuitry 306 by accessing memory 310 to selectively access and load at least one of stimulation programs 316 to therapy delivery circuitry 306. For example, in operation, processing circuitry 304 may access memory 60 to load one of stimulation programs 316 to therapy delivery circuitry 306.

By way of example, processing circuitry 304 may access memory 310 to load one of stimulation programs 316 to control therapy delivery circuitry 306 for delivering the electrical stimulation to patient 102. A clinician or patient 102 may select a particular one of stimulation programs 316 from a list using a programming device, such as a patient programmer or a clinician programmer. Processing circuitry 304 may receive the selection via telemetry circuitry 312. Therapy delivery circuitry 306 delivers the electrical stimulation to patient 102 according to the selected program for an extended period of time, such as minutes or hours while patient 102 is asleep (e.g., as determined from the one or more sensors and/or sensing circuitry 302). For example, processing circuitry 304 may control switch circuitry 308 to couple electrodes 117 to therapy delivery circuitry 306.

Therapy delivery circuitry 306 delivers electrical stimulation according to stimulation parameters. In some examples, therapy delivery circuitry 306 delivers electrical stimulation in the form of electrical pulses. In such examples, relevant stimulation parameters may include a voltage or current pulse amplitude, a pulse rate, a pulse width, a duty cycle, and/or the combination of electrodes 117 that therapy delivery circuitry 306 uses to deliver the stimulation signal. In some examples, therapy delivery circuitry 306 delivers electrical stimulation in the form of continuous waveforms. In such examples, relevant stimulation parameters may include a voltage or current amplitude, a frequency, a shape of the stimulation signal, a duty cycle of the stimulation signal, or the combination of electrodes 117 therapy delivery circuitry 306 uses to deliver the stimulation signal.

In some examples, the stimulation parameters for the stimulation programs 316 may be selected to cause protrusor muscles 120 and/or 122 to an advanced state (e.g., to open-up airway 124). An example range of stimulation parameters for the electrical stimulation that are likely to be effective in treating OSA (e.g., upon application to the hypoglossal nerves to cause protrusor muscles 120, 122 to protrude or upon application to motor points such as motor points 208A, 208B, 210A, and 210B), are as follows:
a. Frequency or pulse rate: between about 20 Hz and about 50 Hz, and possibly lower such as 2 Hz and 4 Hz. In some examples, the minimum target frequency is used which can achieve muscle tetany (e.g., constant contraction) and provide the required force to open the airway.
b. Current Amplitude: between about 0.1 milliamps (mA) and about 20 mA, and more generally from 0.5 mA to 3 mA, and approximately 1.5 mA.
c. Pulse Width: between about 100 microseconds (µs) and about 500 µs. In some examples, a pulse width of 150 µs might be used for reduced power consumption. In some particular examples, the pulse width is approximately 240 µs. In some cases, shorter pulse widths may be used in conjunction with higher current or voltage amplitudes.

Processing circuitry 304 may select stimulation programs 316 for alternating delivery of electrical stimulation between stimulating the left protrusor muscles 120 and/or 122 and the right protrusor muscles 120 and/or 122 on a time basis, such as in examples where two needles and two leads 106 are implanted. In some examples, there may be some overlap in the delivery of electrical stimulation such that for some of amount of time both left and right protrusor muscles 120 and/or 122 are being stimulated. In some examples, there may be a pause in alternating stimulation (e.g., stimulate left protrusor muscles, a time period with no stimulation, then stimulate right protrusor muscles, and so forth). Processing circuitry 304 may also select stimulation programs 316 that select between different combinations of electrodes 117 for stimulating, such as to stimulate different locations of the hypoglossal nerve(s), which may help with fatigue as well as provide more granular control of how much to protrude tongue 118.

In the example of FIG. 3, therapy delivery circuitry 306 drives electrodes 117 of lead 106. Specifically, therapy delivery circuitry 306 delivers electrical stimulation (e.g., regulated current or voltage pulses at pulse rates and pulse widths described above) to tissue of patient 102 via selected electrodes 117A-117D carried by lead 106. A proximal end of lead 106 extends from the housing of IMD 104 and a distal end of lead 106 extends to a target therapy site, e.g., through inner lumen of the needle. Target therapy sites may include one or both hypoglossal nerves and/or motor points 208A, 210A, 208B and/or 210B. Therapy delivery circuitry 306 may deliver electrical stimulation with electrodes on more than one lead and each of the leads may carry one or more electrodes, such as when patient 102 is implanted with two needles and two leads 106 in tongue 118 for stimulating both hypoglossal nerves simultaneously or bilaterally (e.g., one after the other) or both motor points 208A and 208B and/or motor points 210A and 210B. The leads may be configured as an axial lead with ring electrodes or segmented electrodes and/or paddle leads with electrode pads arranged in a two-dimensional array. The electrodes may operate in a bipolar or multipolar configuration with other electrodes, or may operate in a unipolar configuration referenced to an electrode carried by the device housing or "can" of IMD 104.

In some examples, processing circuitry 304 may control therapy delivery circuitry 306 to deliver or terminate the electrical stimulation based on patient input received via telemetry circuitry 312. Telemetry circuitry 312 includes any suitable hardware, firmware, software, or any combination thereof for communicating with another device, such as an external programmer. Under the control of processing circuitry 304, telemetry circuitry 312 may receive downlink telemetry (e.g., patient input) from and send uplink telemetry (e.g., an alert) to a programmer with the aid of an antenna, which may be internal and/or external. Processing circuitry 304 may provide the data to be uplinked to the programmer and the control signals for telemetry circuitry 312 and receive data from telemetry circuitry 312.

Generally, processing circuitry 304 controls telemetry circuitry 312 to exchange information with a medical device programmer and/or another device external to IMD 104. Processing circuitry 304 may transmit operational information and receive stimulation programs or stimulation parameter adjustments via telemetry circuitry 312. Also, in some examples, IMD 104 may communicate with other implanted devices, such as stimulators, control devices, or sensors, via telemetry circuitry 312.

Power source 314 delivers operating power to the components of IMD 104. Power source 314 may include a battery and a power generation circuit to produce the operating power. In some examples, the battery may be rechargeable to allow extended operation. Recharging may be accomplished through proximal inductive interaction between an external charger and an inductive charging coil within IMD 104. In other examples, an external inductive power supply may transcutaneously power IMD 104 whenever electrical stimulation is to occur.

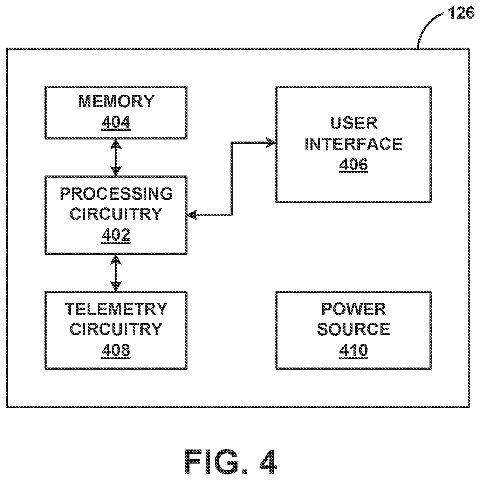

FIG. 4 is a block diagram illustrating an example configuration of an external programmer 130. While programmer 130 may generally be described as a hand-held computing device, programmer 130 may be a notebook computer, a cell phone, or a workstation, for example. As illustrated in FIG. 4, external programmer 130 may include processing circuitry 402, memory 404, user interface 406, telemetry circuitry 408, and power source 410.

In general, programmer 130 comprises any suitable arrangement of hardware, alone or in combination with software and/or firmware, to perform the techniques attributed to programmer 130, and processing circuitry 402, user interface 406, and telemetry circuitry 408 of programmer 130. Examples of processing circuitry 402 may include one or more processors, such as one or more microprocessors, DSPs, ASICs, FPGAs, or any other equivalent integrated or discrete logic circuitry, as well as any combinations of such components. Examples of memory 404 may include RAM, ROM, PROM, EPROM, EEPROM, flash memory, a hard disk, a CD-ROM, comprising executable instructions for causing the one or more processors to perform the actions attributed to them. Moreover, although processing circuitry 402 and telemetry circuitry 408 are described as separate circuitry, in some examples, processing circuitry 402 and telemetry circuitry 408 are functionally integrated. In some examples, processing circuitry 402 and telemetry circuitry 408 correspond to individual hardware units, such as ASICs, DSPs, FPGAs, or other hardware units.

In some examples, memory 404 may further include program information (e.g., stimulation programs) defining the electrical stimulation, similar to those stored in memory 310 of IMD 104. The stimulation programs stored in memory 404 may be downloaded into memory 310 of IMD 104.

User interface 406 may include a button or keypad, lights, a speaker for voice commands, a display, such as a liquid crystal (LCD), light-emitting diode (LED), or cathode ray tube (CRT). In some examples the display may be a touch screen. As discussed in this disclosure, processing circuitry 402 may present and receive information relating to electrical stimulation and resulting therapeutic effects via user interface 406. For example, processing circuitry 402 may receive patient input via user interface 406. The input may be, for example, in the form of pressing a button on a keypad or selecting an icon from a touch screen.

Processing circuitry 402 may also present information to the patient in the form of alerts related to delivery of the electrical stimulation to patient 102 or a caregiver via user interface 406. Although not shown, programmer 130 may additionally or alternatively include a data or network interface to another computing device, to facilitate communication with the other device, and presentation of information relating to the electrical stimulation and therapeutic effects after termination of the electrical stimulation via the other device.

Telemetry circuitry 408 supports wireless communication between IMD 104 and programmer 130 under the control of processing circuitry 402. Telemetry circuitry 408 may also be configured to communicate with another computing device via wireless communication techniques, or direct communication through a wired connection. In some examples, telemetry circuitry 408 may be substantially similar to telemetry circuitry 312 of IMD 104 described above, providing wireless communication via an RF or proximal inductive medium. In some examples, telemetry circuitry 408 may include an antenna, which may take on a variety of forms, such as an internal or external antenna.

Examples of local wireless communication techniques that may be employed to facilitate communication between programmer 130 and another computing device include RF communication according to the 802.11 or Bluetooth specification sets, infrared communication (e.g., according to the IrDA standard), or other standard or proprietary telemetry protocols. In this manner, other external devices may be capable of communicating with programmer 130 without needing to establish a secure wireless connection.

Power source 410 delivers operating power to the components of programmer 130. Power source 410 may include a battery and a power generation circuit to produce the operating power. In some examples, the battery may be rechargeable to allow extended operation.

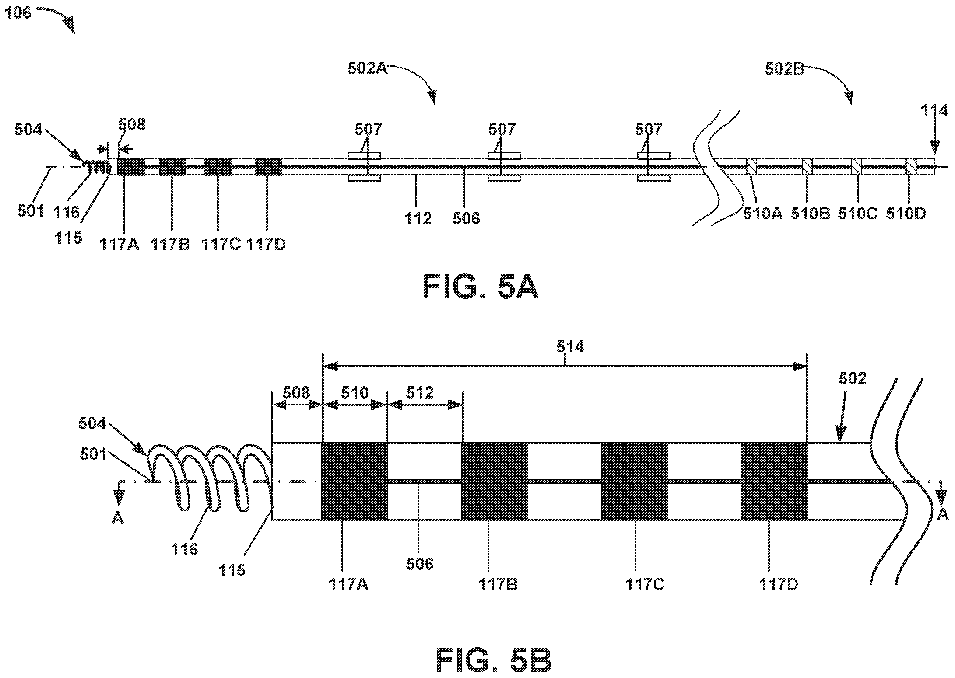

FIG. 5A is a conceptual diagram illustrating an example implantable lead 106 with an fixation element 116. Lead 106 includes elongated body 112 extending along longitudinal axis from distal end 115 to proximal end 114 and defines distal portion 502A and proximal portion 502B.

Distal portion 502A may include one or more electrodes 117A-117D (collectively referred to as "electrodes 117") disposed on lead body 112 and fixation element 116 disposed on lead distal end 115 and extending distally along longitudinal axis 501. Electrodes 117 may be electrically connected to conductor(s) 506 disposed along and/or within lead body 112. At proximal portion 502B, conductor(s) 506 may electrically connects each of electrodes 117 to a corresponding electrical connector of electrical connectors 510A - 510D (collectively referred to as "electrical connectors 510"). Electrical connectors 510 may electrically connect electrodes 117 to IMD 104 (e.g., to switch circuitry 308 of IMD 104).

A proximal end of fixation element 116 may be separated from a distalmost electrode 117A by a distance 508. Fixation element 116 may be separated from distalmost electrode 117A by distance 508 to electrically isolate fixation element 116 from distalmost electrode 117A. Fixation element 116 may extend from lead distal end 115 into a distal tip 504. Distal tip 504 may be configured to penetrate tissue of patient 102 (e.g., tissue of protrusor muscles 120 and/or 122) and to facilitate advancement of fixation element 116 into the tissue of patient 102. Distal tip 504 may include a puncturing tip.

Distal portion 502A may further include a plurality of protrusions 507 disposed on lead body 112 and extending radially away from longitudinal axis 501. Each of protrusions 507 may be configured to engage with tissue of patient 102 and to increase friction between lead 106 and the tissue of patient 102, e.g., to prevent dislodgment of lead 106. In some examples, protrusions 507 may engage the tissue of patient 102, e.g., to prevent rotation of active fixation element 116 within the tissue of patient 102. Protrusions 507 may extend between about 1 mm to about 3 mm away from an outer surface of lead body 112. In some examples, as illustrated in FIG. 5A, protrusions 507 may be proximal to electrodes 117. In some examples, protrusions 507 may be disposed between at least two of electrodes 117.

Although protrusions 507 are illustrated as being proximal to electrodes 117 (e.g., towards IMD 104 and away from distal end), in some examples, the positioning of electrodes 117 and protrusions 507 may be flipped. That is, protrusions 507 may be more distal to electrodes 117. The location of protrusions 507 relative to electrodes 117 is illustrated for ease and should not be considered limiting.

In some examples, lead body 112 may include other protrusions, indentations, creases, or other texturing dispose on the outer surface of lead body 112 and over at least a portion of distal portion 502A and/or between electrodes 117. The texturing may increase friction between the tissue of patient 502 and lead body 112 and may prevent dislodgement of lead 106 within the tissue.

FIG. 5B is a conceptual diagram illustrating distal portion 502A of the example implantable lead 106 of FIG. 5A. Distal portion 502A may include an electrically active length 514 of lead body 112. Electrically active length 514 may correspond to a portion of lead 106 configured to transmit electrical stimulation signals to and/or sense electrical signals from the tissue of patient 102. Electrically active length 514 may extend from a distal end of distalmost electrode 117A to a proximal end of a proximal most electrode (e.g., electrode 117D). While the example leads 106 illustrated in FIGS. 5A and 5B include four electrodes 117, other example leads may include two, three, or five or more electrodes 117.

Each of electrodes 117 may be separated by a distance 512, e.g., to electrically isolate the respective electrode from adjacent electrodes 117. Distalmost electrode 117A is separated from fixation element 116 by distance 508, e.g., to electrically isolate fixation element 116 from electrodes 117. Distance 508 may be between about 1 mm to about 5 mm.

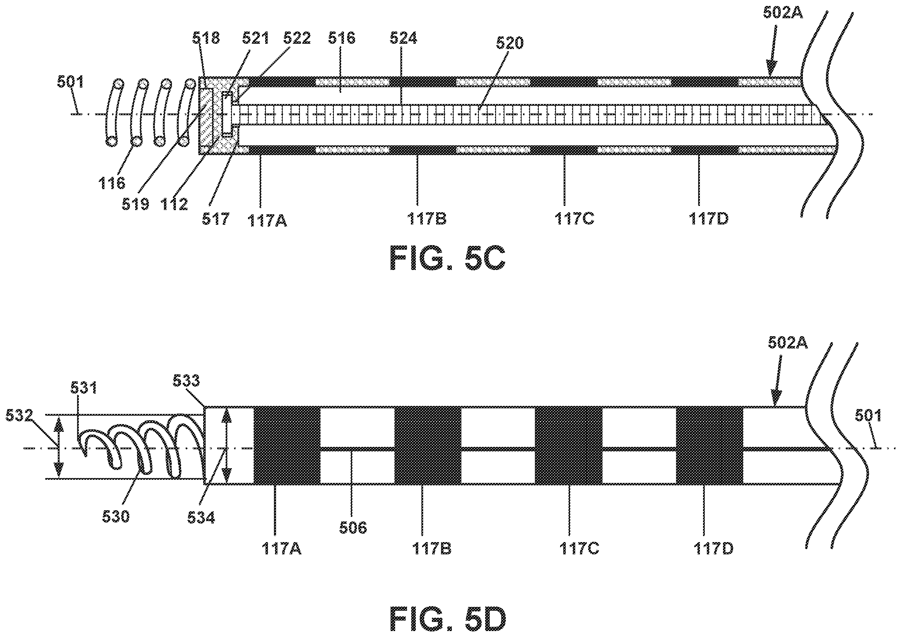

As illustrated in FIGS. 5A and 5B, fixation element 116 may define a helical or spiral shape and terminate in distal tip 504. In other examples, fixation element 116 may have any other shape (e.g., a spiral or helix with another geometric shape including, but is not limited, a triangular shape, a square shape, a pentagonal shape, a hexagonal shape, or the like). Fixation element 116 may have a constant or a variable outer diameter. In some examples, fixation element 116 may have a tapered diameter expanding from distal tip 504 up to a maximum outer diameter at the proximal end (e.g., as described in greater detail with respect to FIG. 5D). Fixation element 116 may have a constant or a variable pitch. In some examples, fixation element 116 may have a pitch of between 2 and 3 mm. Fixation element 116 may have a circular cross-section or may have a cross-section of any other geometric shape (e.g., a triangular cross-section, a pentagonal cross-section, or the like). Fixation element 116 may include one or more biocompatible metallic materials including, but is not limited to, stainless steel, titanium, or platinum).

A maximum diameter of fixation element 116 may be less than or equal to a maximum outer diameter of lead body 112. For example, if lead body 112 has a maximum outer diameter of 6 Fr (about 2 mm), fixation element 116 may have a maximum outer diameter less than or equal to 6 Fr.

FIG. 5C is a conceptual diagram illustrating a cross-sectional view of the distal portion of FIG. 5A, the cross section being taken along line A-A in FIG. 5B. As illustrated in FIG. 5B, line A-A is parallel to longitudinal axis 501. In some examples, as illustrated in FIG. 5C, lead body 112 may be an elongated tube defining lead lumen 516. Lead lumen 516 may extend down at least a portion of lead body 112 and may terminate at lead lumen distal end 517. Distal portion 502A of lead may further define a locking recess 522 extending distally past lead lumen distal end 517 and/or recess 518 on distal end 115. Recess 518 may be configured to receive and/or secure a proximal portion (e.g., base 519) of fixation element 116 within distal portion 502A.

Locking recess 522 may extend away from lead lumen distal end 517 and be configured to receive a distal portion of elongated stylet 520 (also referred to as "locking stylet 520"). The distal portion of elongated stylet 520 may define a locking member 521. The locking member 521 is configured to engage with locking recess 522. Elongated stylet 520 is configured to be disposed within lead lumen 516. In some examples, elongated stylet 520 may be advanced into lead lumen 516 after lead 106 is disposed at the target area within protrusor muscles 120 and/or 122. In other examples, elongated stylet 520 may be secured within locking recess 522 prior to insertion of lead 106 within the introducer and may be advanced to the target area within protrusor muscles 120 and/or 122 alongside lead 106.

When elongated stylet 520 is disposed within locking recess 522, the clinician may rotate elongated stylet 520 to secure elongated stylet 520 within locking recess 522 and prevent proximal movement of elongated stylet out of locking recess 522. Once elongated stylet 520 is secured, the clinician may apply a torque to elongated stylet 520 to cause lead body 112 to rotate about longitudinal axis 501. In other examples, elongated stylet 520 may be configured to engage with locking recess 522 in one or more other manners (e.g., by affixing distal portion of elongated stylet 520 to a protrusion within locking recess 522, by inserting a plurality of protrusions of elongated stylet 520 into a plurality of corresponding locking recesses 522, or the like).

Elongated stylet 520 is configured to transmit the torque to lead body 112 and fixation element 116 via one or more engagement surfaces between elongated stylet 520 and portions of lead body 112 defining locking recess 522. The clinician may apply a torque in a same direction as the winding of fixation element 116, e.g., to puncture tissue of patient 102 via distal tip 504 of fixation element 116 and advance coils of fixation element 116 into the tissue of patient 102. For example, with respect to fixation element 116 illustrated in FIGS. 5A-5C, the clinician may apply a torque in a clockwise direction to advance fixation element 116 into protrusor muscles 120 and/or 122 of patient 102. The clinician may apply a torque in an opposite direction as the winding of fixation element 116 to retract fixation element 116 from within the tissue of patient 102. For examples, with respect to fixation element 116 illustrated in FIGS. 5A-5C, the clinician may apply a torque in a counterclockwise direction to retract fixation element 116 from within protrusor muscles 120 and/or 122 of patient 102.

FIG. 5D is a conceptual diagram illustrating another example distal portion 502A of the example implantable lead 106 of FIG. 5A. As illustrated in FIG. 5D, distal portion 502A may include fixation element 530 that tapers from a proximal end 533 to a distal tip 531.

Fixation element 530 may have similar cross-sections and/or materials as fixation element 116. Fixation element 530 may taper from proximal end 533 with a relatively larger outer diameter to distal tip 531 having a relatively smaller outer diameter. Tapering on the fixation element 530 may facilitate insertion and/or removal of fixation element 530 from tissue of patient (e.g., from within protrusor muscles 120 and/or 122 of patient 102). Fixation element 530 may have a constant pitch from distal tip 531 to proximal end 533. In some examples, fixation element 530 may have a variable pitch from distal tip 531 to proximal end 533. A portion of fixation element 530 at proximal end 533 may have a larger or smaller pitch than a portion of fixation element 530 at distal tip 531.

As illustrated in FIG. 5D, fixation element 530 may have a maximum outer diameter 532 at proximal end 533 and lead body 112 may have an outer diameter 534 at distal portion 502A. Outer diameter 532 is less than or equal to outer diameter 534, e.g., to facilitate navigation of distal portion 502A of lead 106 within the introducer and/or the path in the tissue of patient 102 formed by the needle.

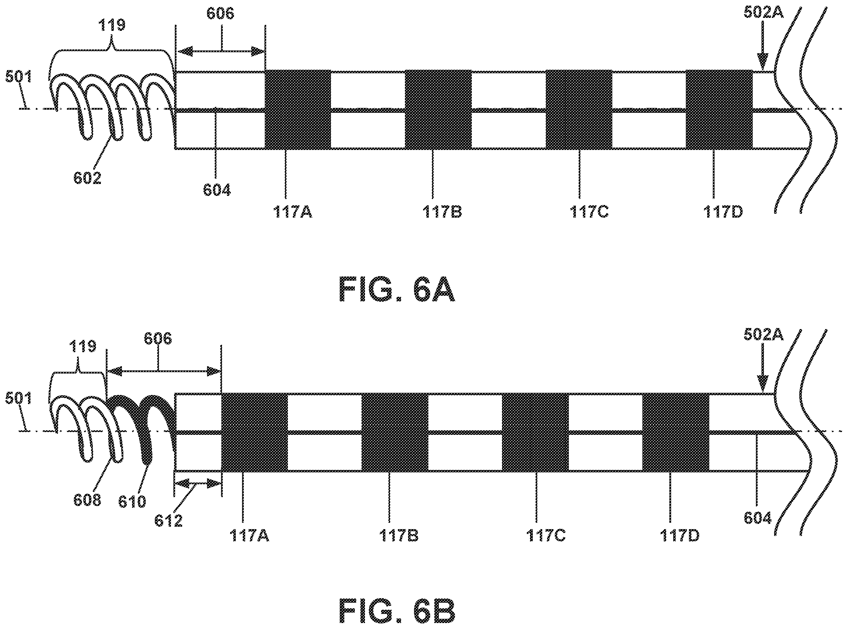

FIG. 6A is a conceptual diagram illustrating an example distal portion 502A of the example implantable lead 106 with an electrically connected fixation element 602. Fixation element 602 may be electrically connected to IMD 104 via conductor 604 extending along the length of lead body 112 to proximal end 114. The dimensions of fixation element 602 may be the same as any other example fixation element described herein (e.g., fixation element 116, fixation element 530). Fixation element 602 may define a distal electrode 119 over at least a part of fixation element 602.

Distal electrode 119 may be configured to deliver electrical stimulation signals to hypoglossal nerve(s) and/or motor points (e.g., motor points 208 and/or 210) of patient 102 and/or sense electrical signals (e.g., evoked electrical signals) from tissue of patient 102, e.g., in a manner similar to any of electrodes 117. Distal electrode 119 may be configured to deliver monopolar electrical signals to a reference electrode disposed on the body of patient 102. In some examples, distal electrode 119 may form an electrical circuit with one or more of electrodes 117 and may transmit multipolar electrical signals (e.g., bipolar electrical signals) to or receive multipolar electrical signals from the one or more of electrodes 117. Distal electrode 119 may be of a greater, shorter, or same length as any of electrodes 117.

A proximal end of fixation element 602 may be separated from distalmost electrode 117A by a distance 606, e.g., to electrically isolate fixation element 602. Distance 606 may be measured between a distal end of distalmost electrode 117A and a proximal end of fixation element 602. Distance 606 may be greater than or equal to distance 508. In some examples, distance 606 may be between about 3 mm to about 5 mm.

FIG. 6B is a conceptual diagram illustrating an example distal portion 50A of the example implantable lead 106 with another example electrically connected fixation element 608. The dimensions of fixation element 608 may be the same as any other example fixation element described herein (e.g., fixation element 116, fixation element 530, fixation element 602). Fixation element 608 may include a proximal insulated portion 610 and an electrically active distal portion defining distal electrode 119. Proximal insulated portion 610 may include an insulative material disposed over fixation element 608.

As illustrated in FIG. 6B, distance 606 may include distance 612 between distalmost electrode 117A and a linear length of insulated portion 610 along longitudinal axis 501. Insulated portion 610 may allow for a reduced distance between the proximal end of fixation element 608 and distalmost electrode 117A while still maintaining distance 606 between distalmost electrode 117A and distal electrode 119.

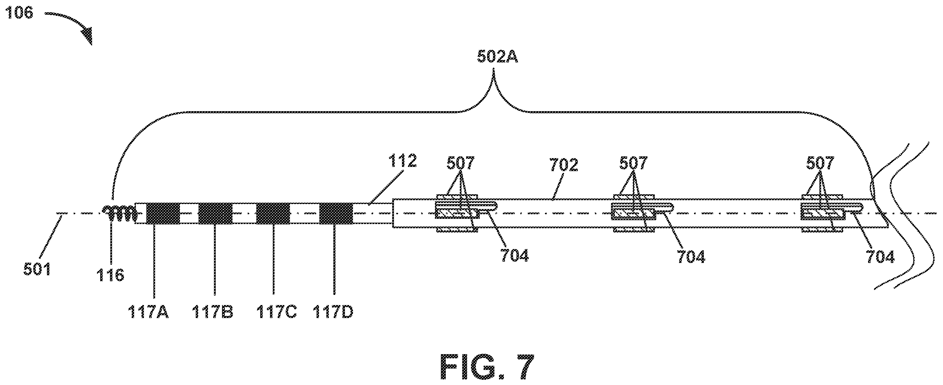

FIG. 7 is a conceptual diagram illustrating the example implantable lead 106 of FIG. 5A with an example guide sheath 702. In some examples, as illustrated in FIG. 7, a clinician may implant and/or remove fixation element 116 from the tissue of patient 102 by applying a torque to distal portion 502A via guide sheath 702 to rotate lead body 112 about longitudinal axis 501. While FIG. 7 illustrates the use of guide sheath 702 with fixation element 116, guide sheath 702 may be used in conjunction with any combination of the examples described above with respect to FIGS. 1-6B.

In some examples, guide sheath 702 is disposed over lead body 112 and within the introducer lumen of the introducer. In some examples, the introducer functions as guide sheath 702. Guide sheath 702 includes a plurality of openings 704, each of openings 704 corresponding to a corresponding protrusion of protrusions 507 and configured to engage with the corresponding protrusion, e.g., to secure guide sheath 702 to lead body 112. Once guide sheath 702 is secured to lead body 112, the clinician may manipulate a proximal portion of guide sheath 702 to position lead body 112 within the tissue of patient 102. The clinician may apply a torque to the proximal portion of guide sheath 702 to advance and/o retract fixation element 116 from within the tissue of patient 102. The clinician may apply the torque in a same direction as the winding of fixation element 116 to advance fixation element 116 into the tissue. The clinician may apply the torque in an opposite direction as the winding of fixation element 116 to retract fixation element 116 from within the tissue.

In the example illustrated in FIG. 7, each of openings 704 includes a first portion configured to retain a corresponding protrusion of protrusions 507 in a restrained configuration and a second portion configured to allow the corresponding protrusion of protrusions 507 to enter and/or exit opening 704. In such examples, guide sheath 702 may be secured to lead body 112 by inserting protrusions 507 into the second portions of openings 704 and rotating guide sheath 702 to secure protrusions 507 into the first portions of openings 704. In other examples, other example protrusions and/or guide sheaths may be used, e.g., to secure the guide sheath to lead body 112 and/or to apply the torque to lead body 112. In some examples, guide sheath 702 may be disposed over lead body 112 prior to insertion of lead 106 into the path formed by the needle and/or the introducer lumen. In some examples, guide sheath 702 may be advanced over lead body 112 and into the introducer lumen after lead 106 is inserted within the introducer lumen.

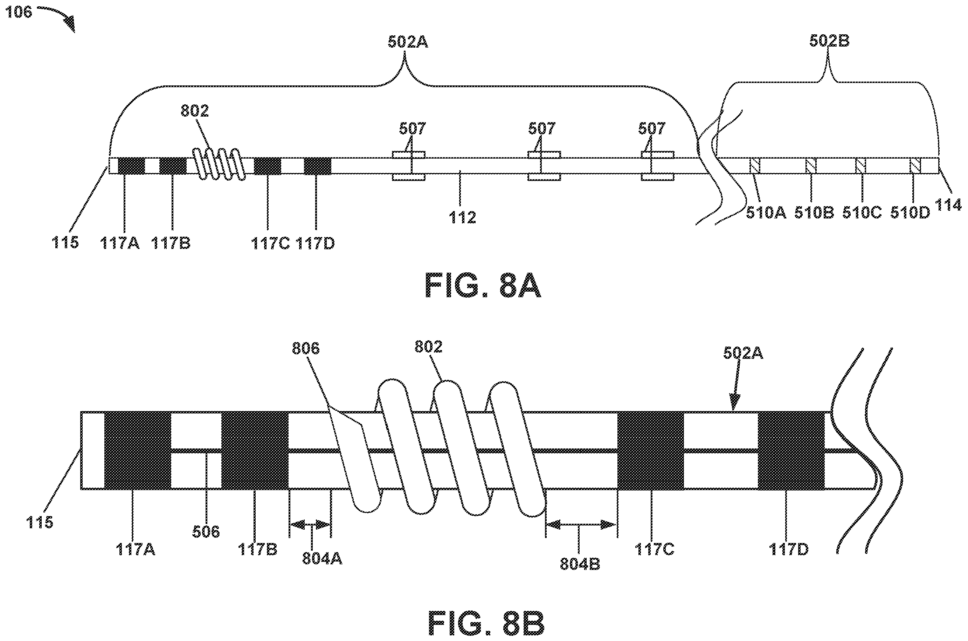

FIG. 8A is a conceptual diagram illustrating an example implantable lead 106 with another example fixation element 802. Fixation element 802 is disposed on lead body 112 and proximal to lead distal end 115. Fixation element 802 may be disposed between two of electrodes 117 or proximal to electrodes 117. When disposed within the path in the tissue of patient 102 formed by the needle, fixation element 802 may puncture and engage with tissue of patient 102 (e.g., of protrusor muscles 120 and/or 122) adjacent to lead body 112

FIG. 8B is a conceptual diagram illustrating the example distal portion 502A of the example implantable lead 106 of FIG. 8A. Fixation element 802 may be separated from adjacent electrodes 117 by distances 804A-804B (collectively referred to as "distances 804"). Distance 804A may be less than, greater than, or the same as distance 804B. Each of distances 804 may be sufficiently large to electrically isolate fixation element 802 from adjacent electrodes 117 (e.g., from electrodes 117B, 117C, as illustrated in FIG. 8B). In some examples, each of distances 804 may be between about 1 mm to about 3 mm.

In some examples, at least a portion of fixation element 802 may be electrically connected to IMD 104 and define a helical electrode (e.g., in a manner similar to distal electrode 119 as illustrated in FIGS. 1 and 6A-6B). In such examples, a first portion (e.g., a distal end and/or a proximal end) of fixation element 802 may be electrically insulated and a second portion of fixation element 802 (e.g., a distal portion, a medial portion, and/or a proximal portion of fixation element 802) may define the helical electrode. In such examples, distances 804 may be between about 3 mm to about 5 mm.

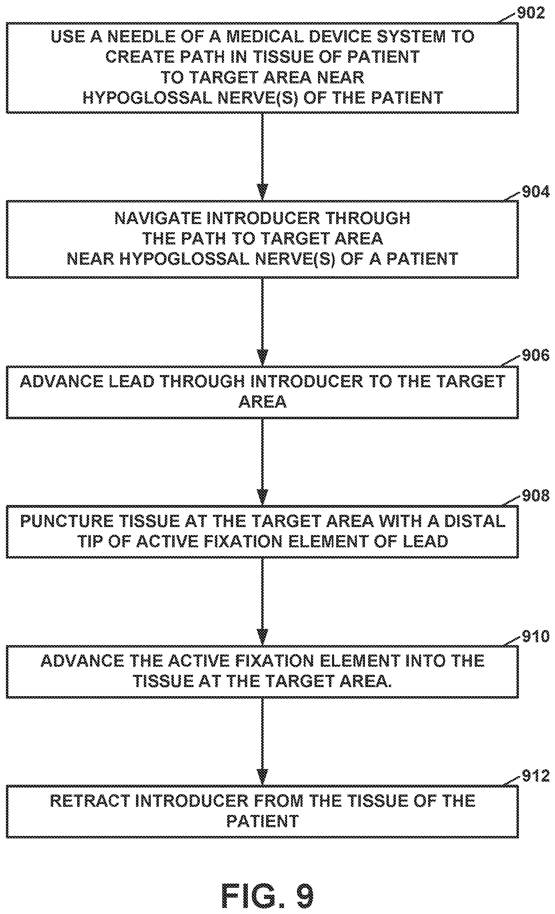

FIG. 9 is a flowchart illustrating an example method of implanting an example implantable lead (e.g., lead 106) near the hypoglossal nerve(s) of patient 102. While the example method illustrated in FIG. 9 is described primarily with reference to fixation element 116, the example method described herein may be used with any other fixation element described above.

A clinician may use a needle of a medical device system 100 to create path in tissue of patient 102 to a target area near hypoglossal nerve(s) of patient 102 (902). The clinician may insert a needle of system 100 through tissue near a chin of patient 102 and through tongue of patient 102 to the target area. The target area may be within one or more of protrusor muscles 120 and/or 122 and near one or more hypoglossal nerve(s) and/or motor points (e.g., motor points 208A, 208B, 210A, and/or 210B). During insertion of the needle into tissue of patient 102, the clinician may insert a trocar into a needle lumen defined by the needle to control an amount of bodily fluids (e.g., blood) in the path in the tissue.

The clinician may deliver test electrical signals (e.g., test stimulation signals) via one or more electrodes on the needle and sense electrical signals from tissue of patient 102 (e.g., evoked electrical signals) via the one or more electrodes on the needle. Based on the sensed electrical signals, the clinician may determine whether the needle is properly positioned within the tissue of patient 102 and readjust the needle within the tissue if the clinician determines that the needle is not properly positioned. The clinician may iteratively deliver test electrical signals to the tissue of patient 102, sense electrical signals from the tissue, and reposition the needle within the tissue until the clinician determines that the needle is properly positioned within the tissue of patient 102.

The clinician may navigate introducer through the path to the target area near hypoglossal nerve(s) of patient 102 (904). The clinician may retract the needle from the path to the target area and then advance the introducer to through the path to the target area. In some examples, the clinician may advance a guide member (e.g., a guidewire) through a needle lumen of the needle to the target area, retract the needle proximally, and advance the introducer along the guide member to the target area. Once the introducer is advanced to the target area, the clinician may retract guide member proximally from the introducer lumen.

The clinician may advance lead 106 through the introducer to the target area near hypoglossal nerve(s) of patient 102 (906). The introducer defines an introducer lumen extending from a distal end of the introducer to a proximal end of the introducer. The clinician may dispose lead 106 into the introducer lumen through the proximal end of the introducer and advance lead 106 along the introducer lumen until electrodes 117 disposed on a distal portion 502A of lead 106 are at the target area near hypoglossal nerve(s) and/or motor points of patient 102. When distal portion 502A of lead 106 are at the target area, distal portion 502A of lead 106 extends distally from the distal end of the introducer.

The clinician may deliver, via one or more of electrodes 117 and/or distal electrodes on fixation element 116 (e.g., distal electrode 119), test stimulation signals to tissue of patient 102 and sense electrical signals from the tissue of patient 102 in response to the test stimulation signals. Based on the sensed electrical signals, the clinician may determine that distal portion 502A is improperly positioned within the tissue of patient 102, reposition distal portion 502A within the tissue of patient 102 to a proper position within the tissue of patient 102.

The clinician may puncture tissue at the target area with distal tip 504 of fixation element 116 of lead 106 (908). The clinician may apply a torque to lead body 112 via an elongated stylet (e.g., elongated stylet 520), a guide sheath (e.g., guide sheath 702), and/or one or more other guide and/or locking mechanisms configured to engage with lead body 112. The applied torque causes lead body 112 to rotate about longitudinal axis 501. The application of the torque to lead body 112 may cause distal tip 504 of fixation element 116 to pierce the tissue at the target area. The clinician may advance fixation element 116 into the tissue at the target area (910). The clinician may continue to apply the torque to lead body 112 to advance fixation element 116 into the tissue until a predetermined portion of fixation element 116 is affixed within the tissue. The predetermined portion of fixation element 116 may be a part of or an entirety of fixation element 116 extending from lead distal end 115.

The clinician may retract introducer from the tissue of patient 102 (912). The clinician may retract introducer proximally away from the tissue of patient 102 when the clinician determines that fixation element 116 is affixed in tissue of patient 102. When the introducer is retracted proximally, a plurality of protrusions 507 disposed on lead body 112 may engage with tissue of patient 102 (e.g., along the path in tissue of patient 102) and provide dislodgement of fixation element 116 from within the tissue of patient 102.

The techniques of this disclosure may be implemented in a wide variety of computing devices, medical devices, or any combination thereof. Any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

It should be noted that system 100, and the techniques described herein, may not be limited to treatment or monitoring of a human patient. In alternative examples, system 100 may be implemented in non-human patients, e.g., primates, canines, equines, pigs, and felines. These other animals may undergo clinical or research therapies that my benefit from the subject matter of this disclosure.

## Claims

1. A system comprising:
an introducer configured to navigate a lead for placement near a hypoglossal nerve of a patient, the introducer comprising an elongated body defining an introducer lumen; and
a lead configured to be disposed within the introducer lumen of the elongated body, the lead comprising:
an elongated shaft defining a longitudinal axis;
one or more electrodes disposed on a distal portion of the shaft, the one or more electrodes being configured to be placed near the hypoglossal nerve and configured to stimulate the hypoglossal nerve for treating obstructive sleep apnea (OSA); and
a fixation element disposed over the distal portion of the elongated shaft, the fixation element comprising:
a distal tip configured to penetrate tissue near the hypoglossal nerve; and
a helix comprising a plurality of coils, the plurality of coils being configured to engage the tissue near the hypoglossal nerve to secure the lead near the hypoglossal nerve,
**characterized in that** lead further comprises a plurality of protrusions extending from an outer surface of the elongated shaft, wherein the plurality of protrusions are configured to engage with the tissue of the patient and prevent rotation of the fixation element within the tissue.

2. The system of claim 1, wherein the one or more electrodes comprises a plurality of electrodes, and wherein the fixation element is disposed between two electrodes of the plurality of electrodes.

3. The system of claim 1, wherein the fixation element is proximal to a proximal-most electrode of the one or more electrodes.

4. The system of claim 1, wherein the fixation element is disposed on a distal end of the elongated shaft.

5. The system of any of claims 1-4, wherein an outer diameter of the fixation element is less than or equal to an outer diameter of the elongated shaft.

6. The system of any of claims 1-5, wherein the helix defines a distal electrode configured to be placed near the hypoglossal nerve and configured to stimulate the hypoglossal nerve for treating OSA or to sense signals.

7. The system of claim 6, wherein the fixation element comprises an insulative material disposed over one or more first coils of the plurality of coils, wherein one or more second coils of the plurality of coils define the distal electrode, and wherein the one or more first coils are proximal to the one or more second coils.

8. The system of any of claims 6 and 7, wherein the distal electrode and an electrode of the one or more electrodes are configured to deliver electrical signals to the hypoglossal nerve by transmitting the electrical signals between the distal electrode and the electrode or are configured to sense signals.

9. The system of any of claims 1-8, wherein the plurality of coils of the helix define a variable pitch or wherein the plurality of coils of the helix define a constant pitch.

10. The system of any of claims 1-9, wherein the helix tapers from a proximal end of the fixation element to the distal tip.

11. The system of any of claims 1-10, further comprising a guide sheath disposed within the introducer lumen and over the elongated shaft of the lead, the guide sheath comprising a plurality of openings, each opening of the plurality of openings being configured to retain a corresponding protrusion of the plurality of protrusions, and wherein when engaged with the plurality of protrusions, the sheath is configured to rotate the lead about the longitudinal axis.

12. The system of any of claims 1-11, wherein the elongated shaft defines a lead lumen, wherein the lead further comprises a locking recess disposed at a distal end of the lead lumen, and wherein the system further comprises:
an elongated stylet disposed within the lead lumen, the elongated stylet comprising a locking member disposed on a distal end of the elongated stylet, the locking member being configured to engage with the locking recess, specifically, wherein when the locking member of the elongated stylet is engaged with the locking recess, the locking member is configured to rotate the lead about the longitudinal axis.

13. The system of any of claims 1-12, wherein the fixation element comprises one or more biocompatible materials, specifically, wherein the one or more biocompatible materials comprises one or more of platinum, stainless steel, or titanium.

14. The system of any of claims 1-13, wherein a proximal end of the fixation element is separated from a distalmost electrode of the one or more electrodes by a predetermined distance.

15. The system of any of claims 1-14, wherein the introducer is configured to be percutaneously inserted into skin of the patient.

## Patentansprüche

1. System, das Folgendes umfasst:
einen Einführer, der dazu ausgelegt ist, eine Leitung zur Platzierung nahe einem hypoglossalen Nerv eines Patienten zu navigieren, wobei der Einführer einen länglichen Körper umfasst, der ein Einführlumen definiert; und
eine Leitung, die dazu ausgelegt ist, innerhalb des Einführlumens des länglichen Körpers angeordnet zu werden, wobei die Leitung Folgendes umfasst:
einen länglichen Schaft, der eine Längsachse definiert; eine oder mehrere Elektroden, die an einem distalen Abschnitt des Schafts angeordnet sind, wobei die eine oder die mehreren Elektroden dazu ausgelegt sind, nahe dem hypoglossalen Nerv platziert zu werden, und dazu ausgelegt sind, den hypoglossalen Nerv zur Behandlung einer obstruktiven Schlafapnoe (OSA) zu stimulieren; und ein Fixierelement, das über dem distalen Abschnitt des länglichen Schafts angeordnet ist, wobei das Fixierelement Folgendes umfasst:
eine distale Spitze, die dazu ausgelegt ist, Gewebe nahe dem hypoglossalen Nerv zu durchdringen; und
eine Helix, die mehrere Windungen umfasst, wobei die mehreren Windungen dazu ausgelegt sind, mit dem Gewebe nahe dem hypoglossalen Nerv in Eingriff zu treten, um die Leitung nahe dem hypoglossalen Nerv zu sichern,
**dadurch gekennzeichnet, dass** die Leitung ferner mehrere Vorsprünge aufweist, die sich aus einer Außenfläche des länglichen Schafts erstrecken, wobei die mehreren Vorsprünge dazu ausgelegt sind, mit dem Gewebe des Patienten in Eingriff zu treten und eine Drehung des Fixierelements innerhalb des Gewebes zu verhindern.

2. System nach Anspruch 1, wobei die eine oder die mehreren Elektroden mehrere Elektroden umfassen und wobei das Fixierelement zwischen zwei Elektroden der mehreren Elektroden angeordnet ist.

3. System nach Anspruch 1, wobei das Fixierelement proximal zu einer am weitesten proximalen Elektrode der einen oder mehreren Elektroden ist.

4. System nach Anspruch 1, wobei das Fixierelement an einem distalen Ende des länglichen Schafts angeordnet ist.

5. System nach einem der Ansprüche 1-4, wobei ein Außendurchmesser des Fixierelements kleiner oder gleich einem Außendurchmesser des länglichen Schafts ist.

6. System nach einem der Ansprüche 1-5, wobei die Helix eine distale Elektrode definiert, die dazu ausgelegt ist, nahe dem hypoglossalen Nerv platziert zu werden, und dazu ausgelegt ist, den hypoglossalen Nerv zur Behandlung von OSA zu stimulieren oder Signale zu erfassen.

7. System nach Anspruch 6, wobei das Fixierelement ein isolierendes Material umfasst, das über einer oder mehreren ersten Wicklungen der mehreren Wicklungen angeordnet ist, wobei eine oder mehrere zweite Wicklungen der mehreren Wicklungen die distale Elektrode definieren und wobei die eine oder die mehreren ersten Wicklungen proximal zu der einen oder den mehreren zweiten Wicklungen sind.

8. System nach einem der Ansprüche 6 und 7, wobei die distale Elektrode und eine Elektrode der einen oder der mehreren Elektroden dazu ausgelegt sind, elektrische Signale an den hypoglossalen Nerv zu liefern, indem die elektrischen Signale zwischen der distalen Elektrode und der Elektrode übertragen werden, oder dazu ausgelegt sind, Signale zu erfassen.

9. System nach einem der Ansprüche 1-8, wobei die mehreren Wicklungen der Helix eine variable Steigung definieren oder wobei die mehreren Wicklungen der Helix eine konstante Steigung definieren.

10. System nach einem der Ansprüche 1-9, wobei sich die Helix von einem proximalen Ende des Fixierelements zu der distalen Spitze verjüngt.

11. System nach einem der Ansprüche 1-10, das ferner eine Führungshülle umfasst, die innerhalb des Einführlumens und über dem länglichen Schaft der Leitung angeordnet ist, wobei die Führungshülle mehrere Öffnungen aufweist, wobei jede Öffnung der mehreren Öffnungen dazu ausgelegt ist, einen entsprechenden Vorsprung der mehreren Vorsprünge zu halten, und wobei die Hülle, wenn sie mit den mehreren Vorsprüngen in Eingriff steht, dazu ausgelegt ist, die Leitung um die Längsachse zu drehen.

12. System nach einem der Ansprüche 1-11, wobei der längliche Schaft ein Leitungslumen definiert, wobei die Leitung ferner eine Verriegelungsausnehmung aufweist, die an einem distalen Ende des Leitungslumens angeordnet ist, und wobei das System ferner Folgendes umfasst:
einen länglichen Mandrin, der innerhalb des Leitungslumens angeordnet ist, wobei der längliche Mandrin ein Verriegelungselement umfasst, das an einem distalen Ende des länglichen Mandrins angeordnet ist, wobei das Verriegelungselement dazu ausgelegt ist, mit der Verriegelungsausnehmung in Eingriff zu treten, wobei insbesondere dann, wenn das Verriegelungselement des länglichen Mandrins mit der Verriegelungsausnehmung in Eingriff steht, das Verriegelungselement dazu ausgelegt ist, die Leitung um die Längsachse zu drehen.

13. System nach einem der Ansprüche 1-12, wobei das Fixierelement ein oder mehrere biokompatible Materialien umfasst, wobei das eine oder die mehreren biokompatiblen Materialien insbesondere eines oder mehrere von Platin, Edelstahl oder Titan umfassen.

14. System nach einem der Ansprüche 1-13, wobei ein proximales Ende des Fixierelements von einer am weitesten distalen Elektrode der einen oder der mehreren Elektroden um einen vorgegebenen Abstand getrennt ist.

15. System nach einem der Ansprüche 1-14, wobei der Einführer dazu ausgelegt ist, perkutan in die Haut des Patienten eingeführt zu werden.

## Revendications

1. Système comprenant :
un introducteur configuré pour guider une sonde en vue d'une mise en place à proximité d'un nerf hypoglosse d'un patient, l'introducteur comprenant un corps allongé définissant une lumière d'introducteur ; et
une sonde configurée pour être disposée à l'intérieur de la lumière d'introducteur du corps allongé, la sonde comprenant :
une tige allongée définissant un axe longitudinal ;
une ou plusieurs électrodes disposées sur une partie distale de la tige, la ou les électrodes étant configurées pour être placées près du nerf hypoglosse et configurées pour stimuler le nerf hypoglosse pour traiter l'apnée obstructive du sommeil (AOS) ; et
un élément de fixation disposé sur la partie distale de la tige allongée, l'élément de fixation comprenant :
une pointe distale configurée pour pénétrer le tissu près du nerf hypoglosse ; et
une hélice comprenant une pluralité de spires, la pluralité de spires étant configurée pour venir en prise avec le tissu près du nerf hypoglosse afin d'assujettir la sonde près du nerf hypoglosse,
**caractérisé en ce que** la sonde comprend en outre une pluralité de protubérances s'étendant depuis une surface extérieure de la tige allongée, la pluralité de protubérances étant configurée pour venir en prise avec le tissu du patient et empêcher la rotation de l'élément de fixation dans le tissu.

2. Système selon la revendication 1, dans lequel la ou les électrodes comprennent une pluralité d'électrodes, et dans lequel l'élément de fixation est disposé entre deux électrodes de la pluralité d'électrodes.

3. Système selon la revendication 1, dans lequel l'élément de fixation est proximal par rapport à une électrode la plus proximale de la ou des électrodes.

4. Système selon la revendication 1, dans lequel l'élément de fixation est disposé sur une extrémité distale de la tige allongée.

5. Système selon l'une quelconque des revendications 1 à 4, dans lequel un diamètre extérieur de l'élément de fixation est inférieur ou égal à un diamètre extérieur de la tige allongée.

6. Système selon l'une quelconque des revendications 1 à 5, dans lequel l'hélice définit une électrode distale configurée pour être placée près du nerf hypoglosse et configurée pour stimuler le nerf hypoglosse pour traiter l'AOS ou pour détecter des signaux.

7. Système selon la revendication 6, dans lequel l'élément de fixation comprend un matériau isolant disposé sur une ou plusieurs premières spires de la pluralité de spires, dans lequel une ou plusieurs deuxièmes spires de la pluralité de spires définissent l'électrode distale, et dans lequel la ou les premières spires sont proximales à la ou aux deuxièmes spires.

8. Système selon l'une quelconque des revendications 6 et 7, dans lequel l'électrode distale et une électrode de la ou des électrodes sont configurées pour délivrer des signaux électriques au nerf hypoglosse en transmettant les signaux électriques entre l'électrode distale et l'électrode ou sont configurées pour détecter des signaux.

9. Système selon l'une quelconque des revendications 1 à 8, dans lequel la pluralité de spires de l'hélice définit un pas variable ou dans lequel la pluralité de spires de l'hélice définit un pas constant.

10. Système selon l'une quelconque des revendications 1 à 9, dans lequel l'hélice se rétrécit depuis une extrémité proximale de l'élément de fixation jusqu'à la pointe distale.

11. Système selon l'une quelconque des revendications 1 à 10, comprenant en outre une gaine de guidage disposée à l'intérieur de la lumière d'introducteur et au-dessus de la tige allongée de la sonde, la gaine de guidage comprenant une pluralité d'ouvertures, chaque ouverture de la pluralité d'ouvertures étant configurée pour retenir une protubérance correspondante de la pluralité de protubérances, et dans lequel lorsqu'elle est en prise avec la pluralité de protubérances, la gaine est configurée pour faire tourner la sonde autour de l'axe longitudinal.

12. Système selon l'une quelconque des revendications 1 à 11, dans lequel la tige allongée définit une lumière de sonde, dans lequel la lumière de sonde comprend en outre un évidement de verrouillage disposé à une extrémité distale de la lumière de sonde, et dans lequel le système comprend en outre :
un stylet allongé disposé à l'intérieur de la lumière de sonde, le stylet allongé comprenant un élément de verrouillage disposé sur une extrémité distale du stylet allongé, l'élément de verrouillage étant configuré pour venir en prise avec l'évidement de verrouillage, en particulier, dans lequel lorsque l'élément de verrouillage du stylet allongé est en prise avec l'évidement de verrouillage, l'élément de verrouillage est configuré pour faire tourner la sonde autour de l'axe longitudinal.

13. Système selon l'une quelconque des revendications 1 à 12, dans lequel l'élément de fixation comprend un ou plusieurs matériaux biocompatibles, spécifiquement, dans lequel le ou les matériaux biocompatibles comprennent un ou plusieurs parmi platine, acier inoxydable et titane.

14. Système selon l'une quelconque des revendications 1 à 13, dans lequel une extrémité proximale de l'élément de fixation est séparée d'une électrode la plus distale de la ou des électrodes par une distance prédéterminée.

15. Système selon l'une quelconque des revendications 1 à 14, dans lequel l'introducteur est configuré pour être inséré par voie percutanée dans la peau du patient.
